# EUROPEAN PATENT APPLICATION

(11) **EP 0 438 310 A1**
(43) Date of publication of application: **24.07.1991**
(21) Application number: 91300362.0
(22) Date of filing: 17.01.1991
(51) Int. Cl.: C12N 15/13, C12P 21/08

(54) **Method for producing recombinant immunoglobuline**

(30) Priority: 19.01.1990 US 467700; 20.12.1990 US 627423
(71) Applicant: MERCK & CO. INC., Rahway New Jersey 07065-0900 (US)
(72) Inventor: Law, Ming-Fan, San Diego, California 92129 (US); Mark, George E., III, Princeton Junction, NJ 08550 (US); Singer, Irwin I., Matawan, NJ 07747 (US); Williamson, Alan R., Westfield, NJ 07090 (US)
(74) Representative: Thompson, John Dr.

(57) **Abstract**

A method for producing recombinant immunoglobulins in which
the unique complementarity determining regions of immunoglobulins from a first animal monoclonal antibody are inserted by recombinant technology into a uniquely selected framework of a second animal, including humans. The recombinant DNA constructs of the invention can be used to transfect cells which will produce the recombinant immunoglobulins.

## Description

### BRIEF DESCRIPTION OF THE DRAWINGS

Figure 1. Primers used to isolate DNA encoding murine kappa light chain variable region and murine IgG2a heavy chain variable region using PCR.

Figure 2. Diagram of antibody structure and PCR products of murine heavy and light chain.

Figure 3. 1B4 amino acid sequence for heavy chain variable region and light chain variable regions 1 and 2 deduced from the nucleic acid sequence of the cloned cDNAs.

Figure 4. Oligodeoxynucleotides used as primers for PCR mutagenesis and amplification of the Rei light chain variable region template so as to graft the CDRs of 1B4 into the Rei light chain variable region.

Figure 5. PCR recombination strategy used in the CDR-grafting of the Rei/1B4 light chain variable region.

Figure 6. Outline of the insertion of light chain variable and constant regions into the light chain expression vector.

Figure 7. Oligodeoxynucleotides used as PCR primers to generate a shortened IgG4 heavy chain. Oligodeoxynucleotide primers used in PCR to re-engineer the thymidine kinase (TK) promotor to facilitate the expression of the neomycin resistance gene. Oligodeoxynucleotide primers used in PCR to clone the IgH enhancer sequence. Oligodeoxynucleotides used as PCR primers to generate a human kappa light chain constant region.

Figure 8. PCR recombination strategy used in the fusing of human signal and intronic sequence to the 1B4 heavy chain variable region.

Figure 9. Oligodeoxynucleotides used as primers for PCR recombination to fuse human signal and intronic sequences onto the 1B4 heavy chain variable region.

Figure 10. Outline of the construction of the neomycin selectable expression vector.

Figure 11. Outline of the insertion of the "chimaeric" 1B4 heavy chain variable region and the shortened human IgG4 heavy chain constant region into the heavy chain expression vector.

Figure 12. Levels of transient expression as determined by trapping ELISA, of the 1B4 chimaeric heavy chain: grafted Rei/1B4 light chain recombinant antibody in CV1, COS 7 and 293 cells.

Figure 13. Competitive binding assay of recombinant "chimaeric"/REI 1B4 (circles) and native murine 1B4 MAb (diamonds) for CD18 on activated human PMNs.

Figure 14. Amino acid sequence composition of the human heavy and light chain variable regions from which framework regions were used to support the murine 1B4 CDRs.

Figure 15. Oligodeoxynucleotides used in the construction of Gal/1B4 heavy chain variable region and Jon/1B4 heavy chain variable region plus those necessary to fuse the human signal and intronic sequences onto these variable regions.

Figure 16. PCR-recombination strategy used in the CDR-grafting of the Gal/1B4 heavy chain and Jon/1B4 heavy chain variable regions.

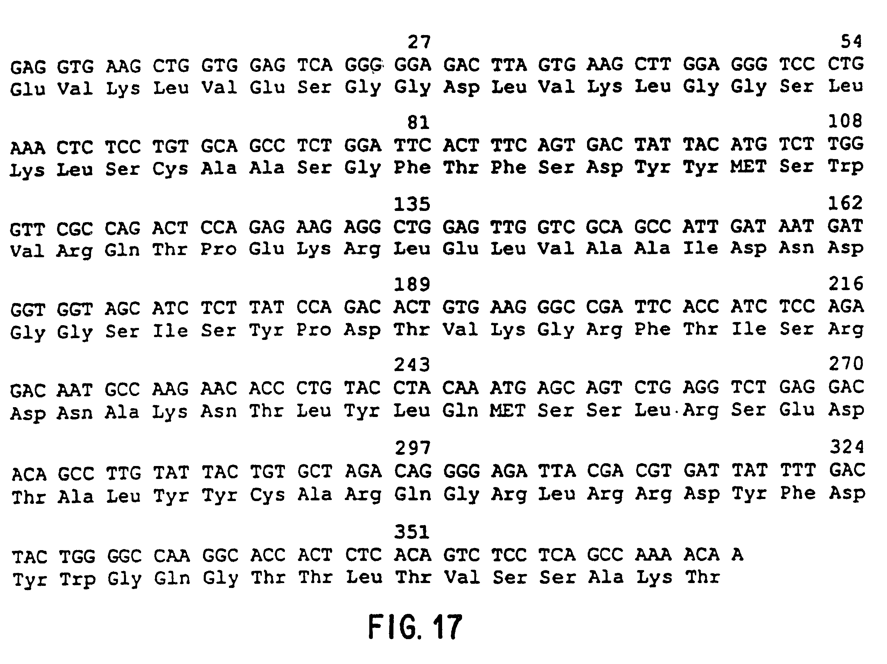

Figure 17. DNA sequence and deduced amino acid sequence determined for murine 1B4 heavy chain variable region.

Figure 18. Outline of the construction of the hygromycin selectable expression vector.

Figure 19. Outline of the insertion of the Gal/1B4 heavy chain and the Jon/1B4 heavy chain variable regions into the heavy chain expression vector containing the shortened IgG4 heavy chain constant region.

Figure 20. Summary of the competitive binding activities of murine MAb 1B4 and recombinant human anti-CD18 antibody constructs.

Figure 21. Oligodeoxynucleotides used in the construction of Len/1B4 light chain variable region plus those necessary to fuse the human signal onto the Len light chain variable region.

Figure 22. PCR-recombination stratagy used in the CDR-grafting of the Len/1B4 light chain variable region.

Figure 23. Outline of the insertion of the Len/1B4 light chain variable region into an interdemediate vector followed by its insertion into the light chain expression vector.

Figure 24. DNA sequence and deduced amino acid sequence determined for murine 1B4 light chain-1 variable region.

Figure 25. DNA sequence and deduced amino acid sequence determined for murine 1B4 light chain-2 variable region.

Figure 26. Oligodeoxynucleotides used in the construction of Gal-m1/1B4 (mutant) heavy chain variable region plus those necessary to fuse the human signal onto the Gal-m1 heavy chain variable region.

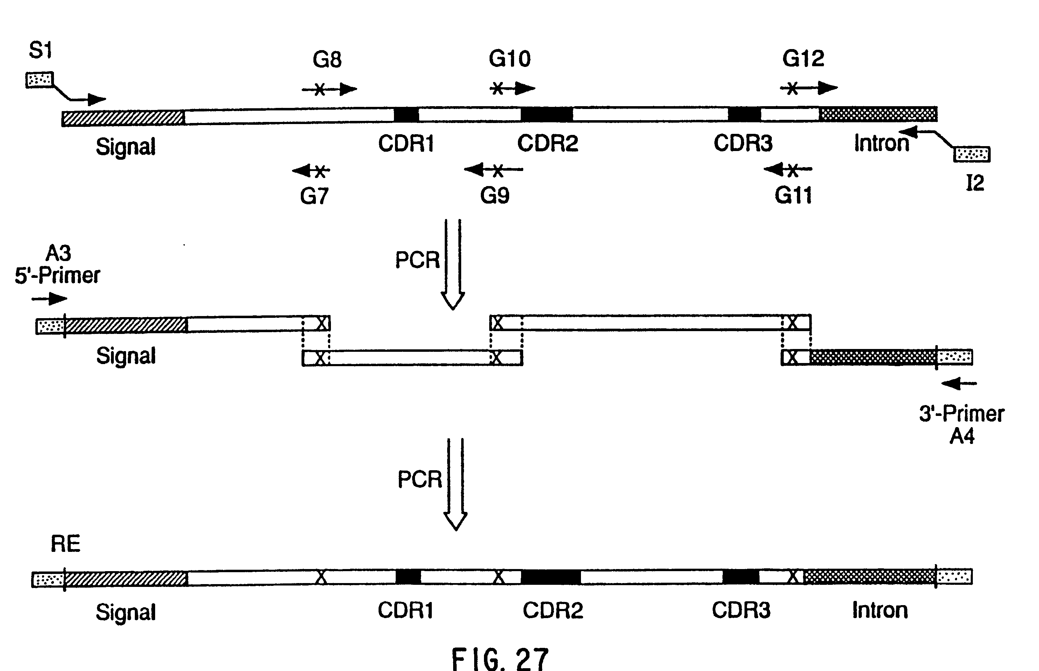

Figure 27. PCR-recombination strategy used in the CDR-grafting of the Gal-m1/1B4 (mutant) heavy chain variable region.

Figure 28. Competitive binding assay of native murine 1B4 (diaminds) and Gal/Rei humanized 1B4 (circles).

Figure 29. Competitive binding assay of New/Rei recombinant h1B4 (closed diamonds) and Gal/Rei recombinant h1B4 (open diamonds).

Figure 30. Effects of native murine 1B4 (diamonds) and Gal/Rei recombinant humanized 1B4 (circles) on attachment of human PMNs to human unbilical vein endothelial cell monolayers in vitro.

Figure 31. Comparison of Gal/Rei h1B4 and m1B4 in in vitro functional assays.

Figure 32. Immunofluorescence microscopic localization of m1B4 and Gal/Rei h1B4 staining in 5 µm forzen sections of rabbit tissues.

Figure 33. Double label immunofluorescence microscopic localization of Gal/Rei h1B4 and m1B4 in rabbit bone marrow cells.

Figure 34. Double label immunoelectron microscopic localization of Gal/Rei h1B4 and m1B4 in specific granules of human PMNs.

Figure 35. Dose-dependet inhibition by of m1B4 and Gal/Rei h1B4 of C5a (100 pmol)-induced PMN accumulation in rabbit skin.

Figure 36. Dose-dependent inhibition by m1B4 and Gal/Rei h1B4 of C5a (100 pmol)-induced plasma extravasation in rabbit skin.

Figure 37. Outline of the construction of expression system p8962 capable of producing large quantities of recombinant CDR-grafted 1B4 antibodies.

Figure 38. Outline of the construction of expression systems p8968 and p8969 capable of producing large quantities of recombinant CDR-grafted 1B4 antibodies.

### BACKGROUND OF THE INVENTION

Murine derived monoclonal antibodies have been utilized as diagnostic and therapeutic agents for numerous human pathologic conditions including acute inflammatory responses associated with numerous diseases. Administration of murine derived monoclonal antibodies (mMAbs) as therapeutic agents in man has been severely limited by the development of antibody within the recipient to the mouse antigens of the murine derived monoclonal antibody. In attempts to circumvent this outcome mMAbs have been restructured by recombinant DNA technology in such a way as to decrease their immunogenicity in humans. Immunoglobulins are well defined both chemically and biologically with the general structures illustrated in Molecular Cell Biology, Darnell, Lodish, and Baltimore, Eds., Scientific American Books, Inc., W.H. Freeman, New York, NY (1986). Initially, this involved the construction of chimaeric antibodies, Morrison et al., Proc. Natl. Acad. Sci. USA 81: 6851-6855 (1984). Recombinant technology was employed to replace the murine heavy and light chain constant regions with corresponding human constant regions. Upon expression, such interspecies antibody chimaeras yielded molecules with the antigen binding specificities of the parent murine antibody. The following references generally describe chimaeric antibody technology: Lobuglio et al., Proc. Natl. Acad. Sci. USA 86: 4220-4224 (1989); United States Patent 4,816,567; PCT International Publication No. WO 87/02671, published May 7,1987; European Patent Publication No. 255,694, published February 10, 1988; European Patent Publication No. 274,394, published July 13, 1988; European Patent Publication No. 323,806, published July 12, 1989; PCT International Publication No. W0/89/00999, published February 9, 1989; European Patent Publication No. 327,000, published August 9, 1989; European Patent Publication No. 328,404, published August 16, 1989; and European Patent Publication No. 332,424, published September 13, 1989.

The immunogenicity of chimaeric antibodies can be further reduced by grafting rodent hypervariable regions into the variable region frameworks of human light and heavy chains, Jones et al., Nature 321: 522-525 (1986). These hypervariable regions have also been termed complementarity determining regions (CDR). The technique involves the substitution or recombinant grafting of antigen-specific murine CDR sequences for those existent within "generic" human heavy and light chain variable regions, European Patent Publication No. 239,400, published September 30, 1987. In this approach, little, if any, concern is shown for the variable region frameworks (FRs) within which the murine CDRs are placed. The instant invention illustrates that appropriate supportive structures for the CDRs are vital not only for the assembly of the functional antibody molecules but also for the production of antibody molecules with avidities which allow for the administration of therapeutic doses (about 0.1-1mg/kg).

Recent studies by Queen et al., Proc. Natl. Acad. Sci. USA 86: 10029-10033 (1989), have shown the CDRs from a murine anti-Tac monoclonal antibody can be grafted into a human framework. The human framework variable regions were chosen to maximize identity with the murine sequence. The authors also utilized a computer model of the mMAb to identify several amino acids which, while outside the CDRs, are close enough to interact with the CDRs or antigen. These residues were mutated to the residue found in the murine sequence. The grafted anti-Tac antibody had an affinity for the antigen which was only about 1/3 that of the murine anti-tac mMAb and maintenance of the human character of this antibody was problematic.

Leukocyte infiltration into an inflammatory site is dependent on the adhesion of the leukocytes to the endothelium prior to extravasation. The rapid binding of polymorhonuclear leukocytes (PMN) to the endothelium and diapedesis occurs within minutes after the introduction of a chemotactic stimulus in tissue, Cybulski et al., Am. J. Pathol. 124: 367 (1986). This rapid extravasation appears to depend on the response of the PMNs to chemoattractants and on the presence of the CD11/CD18 family of glycoproteins on the leukocyte surface. The family of glycoproteins associated with PMNs are termed leukocyte integrins and include LFA-1 (CD11a/CD18), Mac-1 (CD11b/CD18) and p150,95 (CD11c/CD18). Each of these heterodimers has a unique alpha chain (CD11 a, b, c) and an invariant beta-2 chain (CD18). Stimulation of PMNs with various chemotactic factors causes increased expression of leukocyte integrins (CD11b/CD18) fostering strong adhesion to unstimulated endothelium in vitro, Harlan, Blood 65: 513 (1985), and essentially all of the chemoattractant-induced adhesion is inhibited by treating the PMNs with mMAbs specifically reactive with the CD11/CD18 complex, Harlan et al., Blood 66: 167 (1985); Zimmerman and McIntyre J. Clin. Invest. 81: 531 (1988); Smith et al., J. Clin. Invest. 82: 1746 (1988); and Lo et al., J. Exp. Med. 169: 1779 (1989). Polymorphonuclear leukocytes from patients with leukocyte adhesion deficiency (LAD) fail to express CD18 and fail to bind unstimulated endothelium in vitro, Harlan et al., Blood 66: 167 (1985); Lo et al., J. Exp. Med. 169: 1779 (1989).

Murine hybridomas producing monoclonal antibodies reactive with the beta chain common to the Mac-1, LFA-1 and the p150,95 integrins have been described. The mMAbs are designated 1B4, 60.3, TS1/18, H52 and ATCC TIB 218. The 1B4 is an IgG2a antibody and was prepared by Wright et al., Proc. Natl. Acad. Sci. USA 80: 5699-5703 (1983), the 60.3 is also IgG2a and was prepared by Beatty et al., J. Immunol. 131:2913-2918 (1983), TS1/18 is an IgG1 antibody and was prepared by Sanchez-Madrid et al., J. Exp. Med. 158: 1785-1803 (1983), H52, a MAb against beta 2 (CD18) was prepared by Hildreth and Orentas, Science 244: 1075-1078 (1989) and ATCC TIB 218, a IgG2a kappa prepared by Springer et al., J. Exp. Med. 158: 586-602 (1983). These antibodies appear to be functionally equivalent and cross-react with the beta-2 chain found on human, sheep pig, rabbit, and dog leukocytes but not with the beta-2 chain found on murine and rat leukocytes.

### SUMMARY OF THE INVENTION

A method for producing recombinant immunoglobulins in which the unique complementarity determining regions of immunoglobulins from a first animal monoclonal antibody are inserted by recombinant technology into a uniquely selected framework of a second animal, including humans. The recombinant DNA constructs of the invention can be used to transfect cells which will produce the recombinant immunoglobulins.

### OBJECT OF THE INVENTION

It is accordingly, an object of the present invention to provide novel DNA sequences for the complementarity determining regions of murine heavy and light chain monoclonal antibody. Another object of the invention is to provide novel DNA sequences for the complementarity determining regions of murine heavy and light chain monoclonal antibody that immunologically binds to the CD18 integrin or antigen of leukocytes. A further object is to provide novel DNA sequences for recombinant animal antibody. Another object is to provide a vector containing the DNA sequence for recombinant animal antibody. Another object is to provide a mammalian host transformed with a vector containing the DNA sequence for recombinant animal antibody. It is a further object that the animal recombinant antibody be human recombinant antibody. A further objective is to provide recombinant human immunoglobulin that binds to leukocyte integrin. Another object is to provide a process for making recombinant human immunoglobulin. A further object is to provide a process for producing recombinant immunoglobulins.

### DETAILED DESCRIPTION OF THE INVENTION

The present invention relates to methods and means for the construction and expression of unique recombinant derived antibody in which complementarity determining regions (CDRs) from a first animal monoclonal antibody of defined specificity are inserted into a second animal, including man, variable heavy and light chain frameworks which show a high degree of sequence similarity with the frameworks of the first animal and present the CDRs in the appropriate configuration to react with the appropriate antigen or ligand. The insertion or grafting is carried out by processes well known in the biotechnical arts, primarily recombinant DNA technology. The unique frameworks (FRs) are selected for their structural compatibility and sequence similarity with the first animal frameworks. This preselection is dependent on one or more of the following criteria: (i) sequence matching to all known human heavy chain variable (V_{H}) and light chain variable (V_{L}) framework sequences with the framework sequences of the animal monoclonal antibody from which the CDRs have been removed; (ii) sequence matching as described in (i), but with significant attention paied to interspecies matching of the non-surface exposed amino acid residues; (iii) tertiary and quaternary structural model of human framework sequences with CDRs in place for comparison with models of the original animal monoclonal antibody; and (iv) screening of human genomic DNA with DNA probes corresponding to framework sequences in chosen animal monoclonal antibody. These criteria and the following procedures are used to prepare recombinant DNA sequences which incorporate the CDRs of animal mMAb, both light and heavy chains, into human frameworks that can then be used to transfect mammalian cells for the expression of recombinant human antibody with the antigen specificity of the animal monoclonal antibody.

The present invention further comprises a method for constructing and expressing the altered antibody comprising: (i) mutagenesis and assembly of variable region domains including CDRs and FRs regions; (ii) preparation of an expression vector including at least one variable region which upon transfection into cells results in the secretion of protein sufficient for avidity and specificity determinations; and (ii) co-amplification of heavy and light chain expression vectors in appropriate cell lines.

The present invention provides recombinant methods for incorporating CDRs from animal monoclonal antibodies into human immunoglobulin frameworks so that the resulting recombinant human antibody will be either weakly immunogenic or non-immunogenic when administered to humans. Preferably the recombinant immunoglobulins will be recognized as self proteins when administered for threapeutic purposes. This method of "humanization" will render the recombinant antibodies useful as therapeutic agents because they will be either weakly immunogenic or non-immunogenic when administered to humans. The invention is further contemplated to include the recombinant conversion of any animal monoclonal antibody into a recombinant human monoclonal antibody providing that a suitable framework region can be identified (as described below). It is intended that the present invention include the nucleotide and amino acid sequences of the murine CDR regions and the human framework regions either separately or combined as a light or heavy chain or an intact immunoglobulin and any conservatively modified varients thereof. The animal monoclonals may include, but are not limited to, those murine monoclonal antibodies described by VanVoorhis et al., J. Exp. Med. 158: 126-145 (1983) which bind to human leukocytes and the appropriate mMAbs produced by hybridomas deposited in the Hybridoma Cell Bank maintained by the American Type Culture Collection (ATCC) and described in the ATCC Catalog of Cell Lines & Hybridomas, No. 6, 1988.

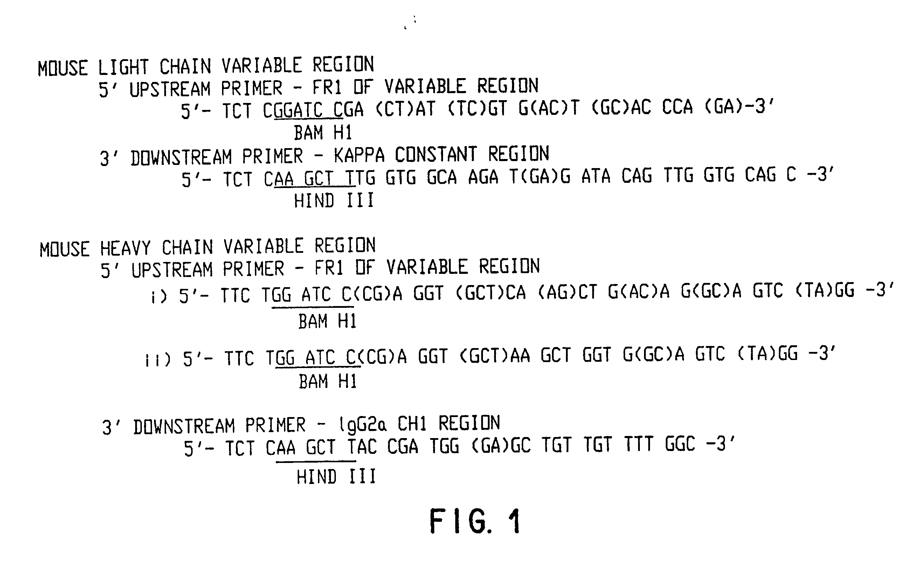

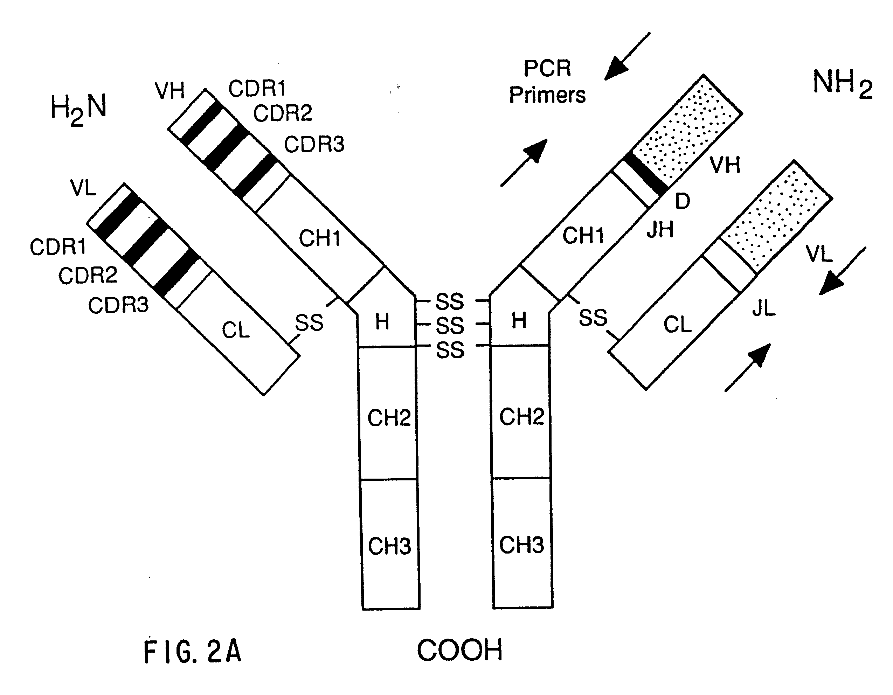

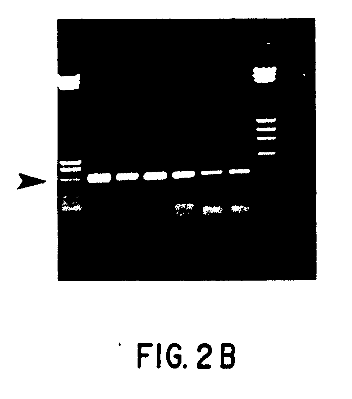

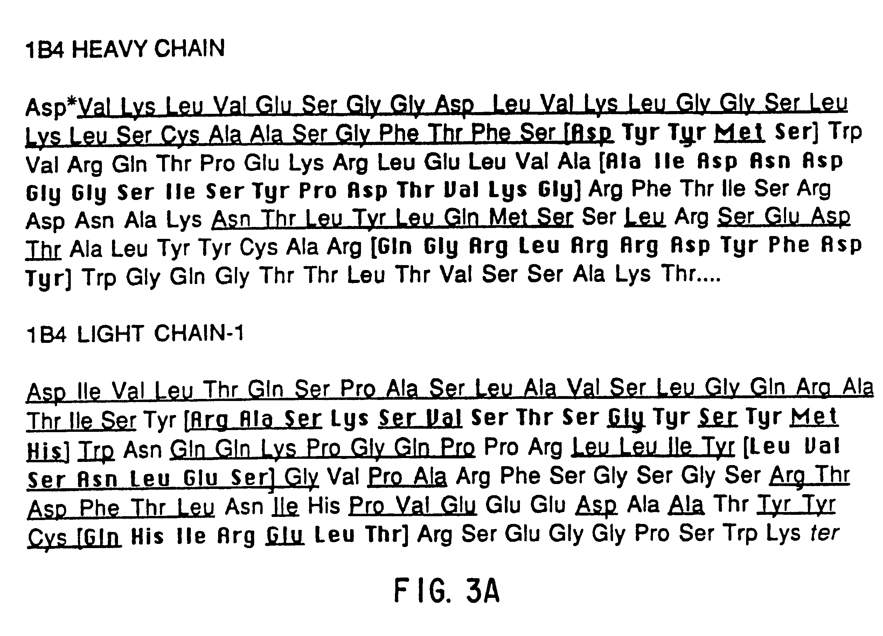

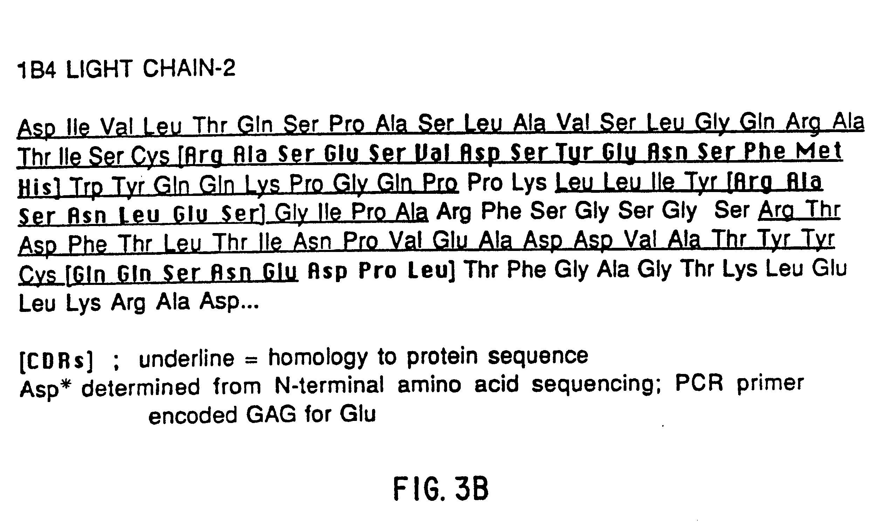

The CDR sequences from the animal monoclonal antibody are derived as follows. Total RNA is extracted from the murine hybridomas, for example the 1B4 myeloma cells described by Wright et al., Proc. Natl. Acad. Sci. USA 80: 5699-5703 (1983), the 60.3 cells described by Beatty et al., J. Immunol. 131: 2913-2918 (1983), the TS1/18 cells described by Sanchez-Madrid et al., J. Exp. Med. 158: 1785-1803 (1983), and other anti-CD18 or CD11 monoclonal antibodies and hybridomas as described in Leukocyte Typing III, Springer-Verlag, New York (1988), using standard methods involving cellular solubilization with guanidinium isothiocyanate (Chirgwin et al., Biochem. 18: 5294-5299[1979]). The murine 1B4 mMAb will be used as the primary example of animal MAb that can be "humanized" by the unique process being disclosed. The invention is intended to include the conversion of any animal immunoglobulin to a human immunoglobulin. It is further intended that human immunoglobulin (Ig) can contain either kappa or lambda light chains or be one of any of the following heavy chain isotypes (alpha, delta, epislon, gamma and mu). Pairs of degenerate oligodeoxynucleotide primers (Figure 1) representing sequences within framework 1 of the murine kappa light chain variable region and light chain constant domain, or those within framework 1 of the murine IgG2a heavy chain variable region and heavy chain constant CH1 domain are synthesized on an Applied Biosystem 381A DNA synthesizer, removed from the resin by treatment with concentrated NH₄OH and desalted on a NAP-5 column eluted with H₂O. Total RNA, about 2 µg, is reverse transcribed for about 30 min at about 42° C using Moloney MLV reverse transcriptase, about 200 units (BRL), and about 10 pmoles of the constant region complementary strand primers for either the heavy or light chain. The reverse transcriptase is heat inactivated, about 95° C for about 5 min, and the reactions are made to contain in about 100 µl of PCR buffer about 50 pmoles of each of the paired primers and and 25 units of Taq polymerase. About 45 cycles of amplification (2′, 94°C; 2′, 55°C; 2′ 72°C) are followed by gel purification of the anticipated 400+ base pair (bp) DNA fragments (Figure 2). Prior to subcloning those DNAs into a blunt-ended intermediate plasmid such as pSP72 (Promega) they are terminally phosphorylated using T4 polynucleotide kinase. Frozen competent E.coli were thawed on ice and 100 µl aliquots were distributed into wet ice chilled polypropylene tubes. DNA (1-10 ng) from the ligation mixture was dispensed with aggitation into these tubes and the mixture was incubated on ice for 30 minutes. The E. coli cells were heat-shocked by incubation at 42° C for 45 seconds, then chilled for 2 minutes on ice. Room temperature S.O.C. (Hanahan, D., J.Mol. Biol. 166: 557, 1983) was added and the cultures were shaken at 225 RPM at 37°C for 60 minutes. Aliquots of the cultures were spread on LB agar plates containing 100 µg/mL ampicillin and these plates were incubated overnight at 37°C to allow for colony growth. Multiple clones representing these PCR amplified sequences are grown and submitted to DNA sequence determinations using Sequenase® and T7 and SP6 specific sequencing primers. A unique DNA sequence representing a murine IgG2a heavy chain variable region is obtained, but two kappa light chain variable regions are represented within the cloned population (Figure 3). To distinguish which sequence belongs to the 1B4 mMAb, the 1B4 mMAb is reduced with dithiothreitol (DTT) and purified heavy and light chains are subjected to N-terminal amino acid sequencing using the Applied Biosystems 477A sequencer. Tryptic and cyanogen bromide digested peptides are also sequenced.

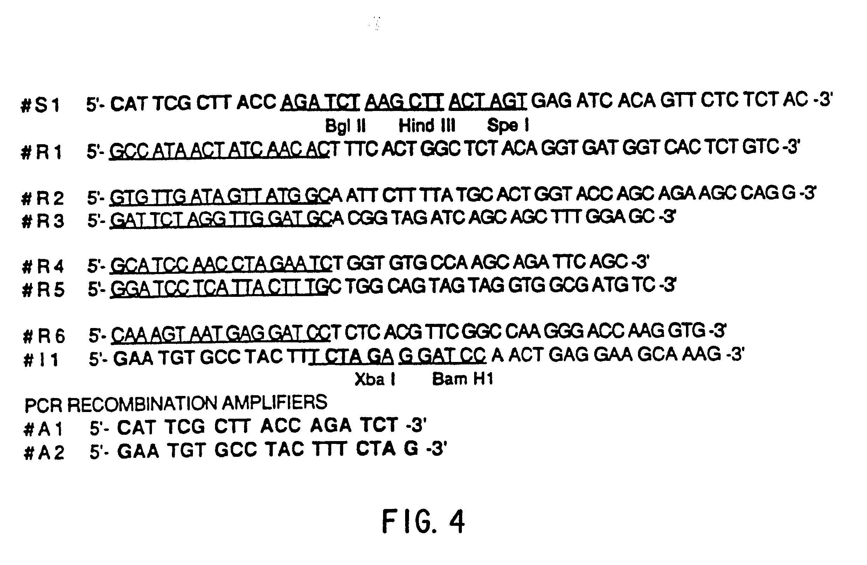

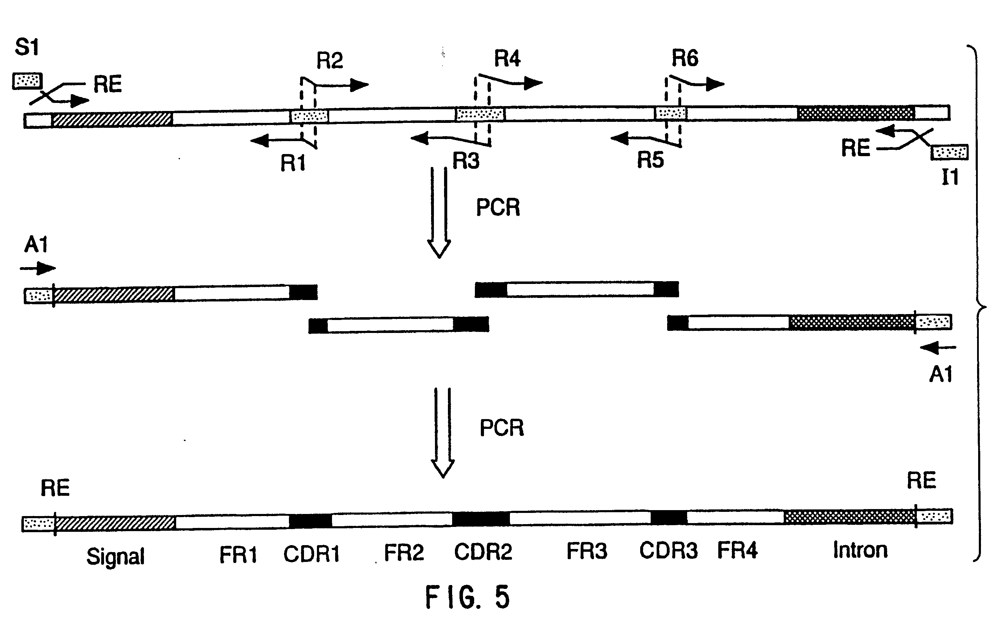

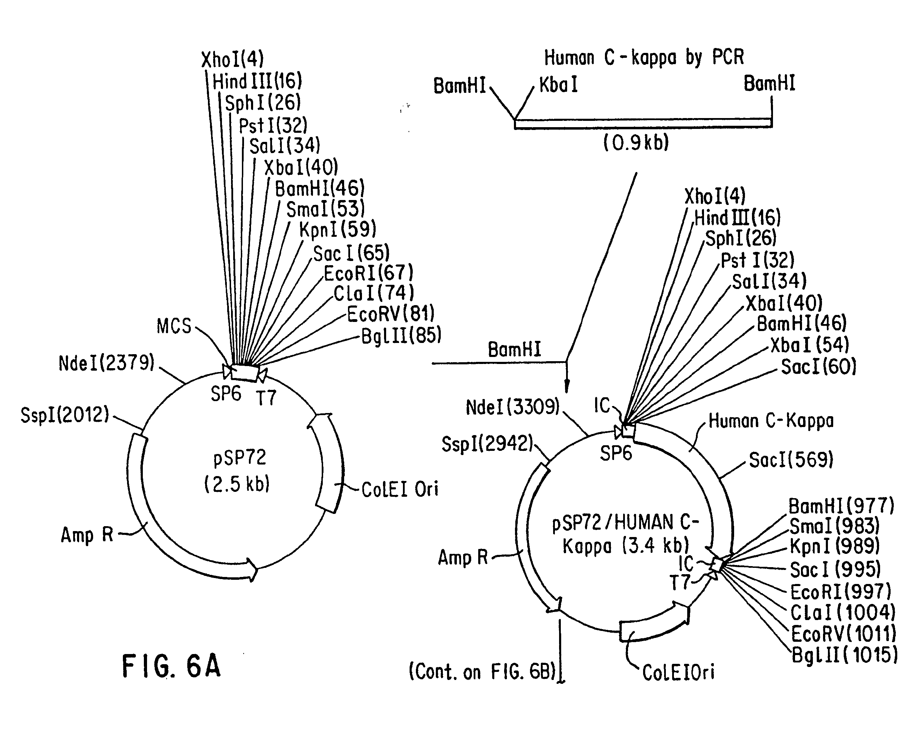

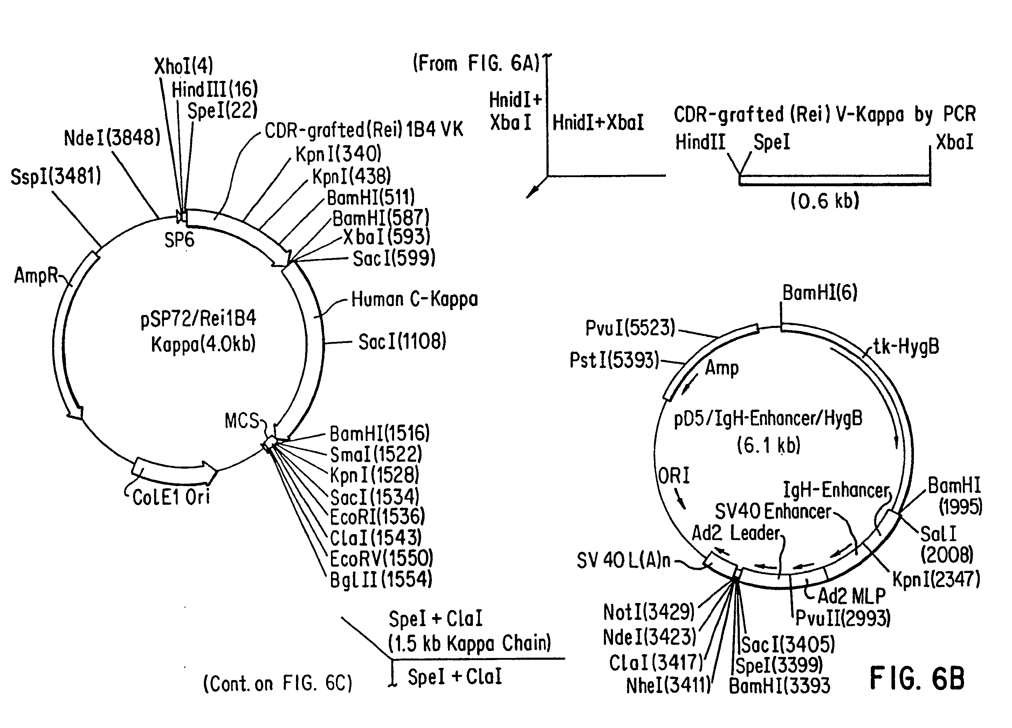

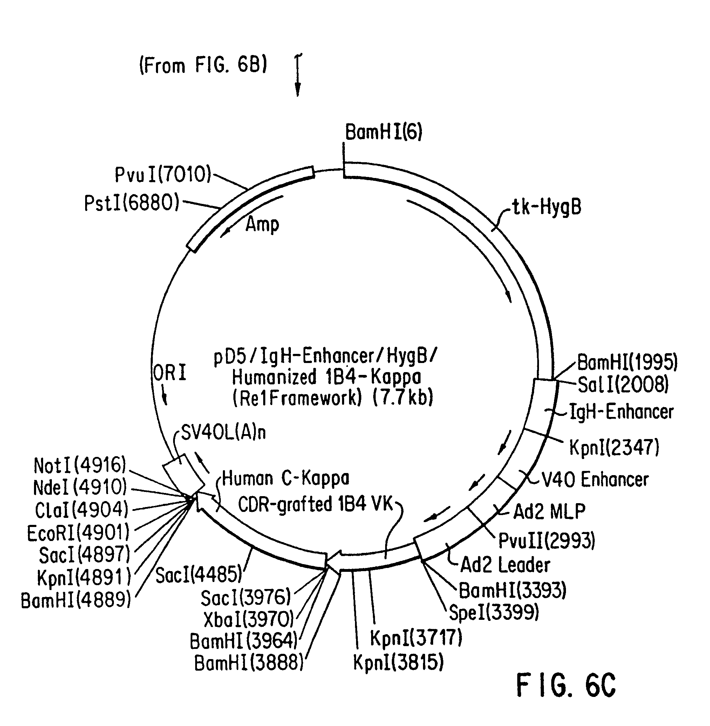

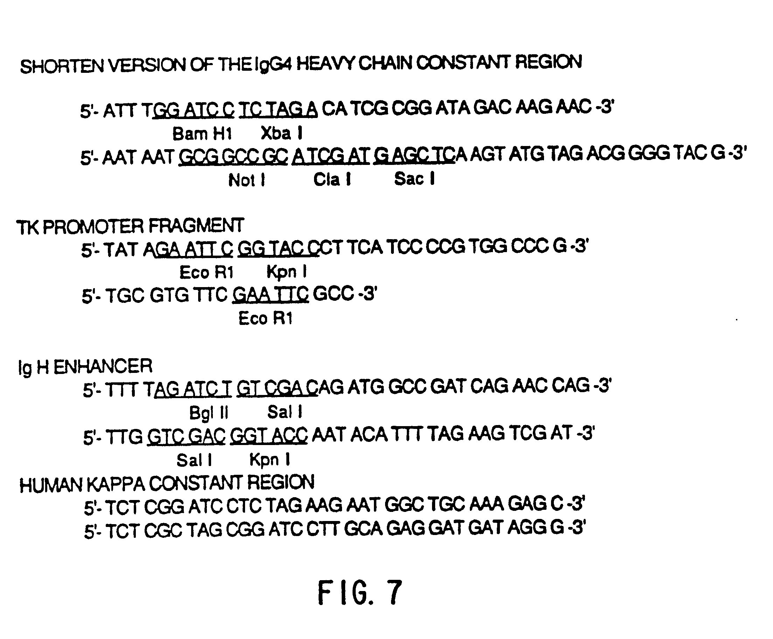

Replacement of human variable region CDRs with those unique to mMAb 1B4 is accomplished utilizing the following unique processes. An appropriate human framework is determined utilizing the criteria discussed above. A light chain variable region framework such as the REI framework (Orlandi, et al., Proc. Natl. Acad. Sci. USA 86: 3833-3837[1989]; Riechmann et al., Nature 332: 323-327[1988]; European Patnet Application, Publication No. 239,400), with its leader and 3′ intronic sequences, is subcloned into the intermediate vector pGEM3Z (Promega). About eight oligodeoxynucleotide primers (Figure 4) are synthesized representing the primers necessary to generate by polymerase chain reaction (PCR) amplification four DNA fragments. Incorporated into all but the terminal oligodeoxynucleotide primers were those sequences corresponding to MAb 1B4 light chain CDRs and at least 15 bases of 5′- terminal complementarity (see Figure 5). The appropriate primer pair, about 50 pmole each, was combined with about 10 ng of plasmid DNA representing the REI framework, about 2.5 units of Taq DNA polymerase and about thirty (30) cycles of PCR amplification ensued (cycle periods, as above). The products of the four reactions, purified by agarose gel electrophoresis, are combined, about 10 ng of each DNA fragment, along with terminal oligodeoxynucleotide primers (Figure 4) and Taq DNA polymerase and the combined fragments were PCR amplified (see Figure 5). Following restriction endonuclease digestion with HindIII and XbaI the amplified DNA is purified by agarose gel electrophoresis and subcloned into compatible sites of an intermediate vector pSP72 (Promega) which contains the human kappa light chain constant region (see Figure 6). Genomic DNA, about 1 µg, purified from a human B cell line (GM0108A: NIGMS Human Genetic Mutant Cell Repository, Institute for Medical Research, Camden, NJ) is used as a template for PCR amplification (Figure 7) of about a 920 base pair fragment containing the splice acceptor for the kappa light chain constant domain, the exon and a portion of its 3′-untranslated region. The PCR product is purified by agarose gel electrophoresis, digested with BamH1 endonuclease, and subcloned into pSP72 previously linearized with BamH1. The individual clones representing the pSP72 intermediate vector containing both the 1B4 grafted variable region derived from REI and the human kappa constant region derived by PCR amplification of human DNA are used to determine the DNA sequence of the grafted light chain variable region.

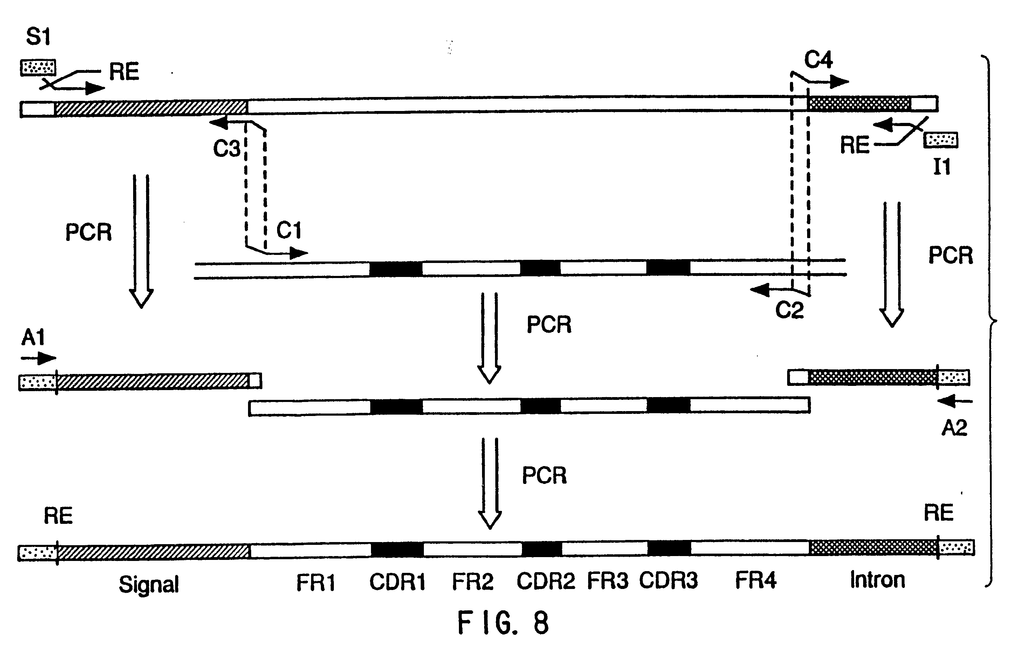

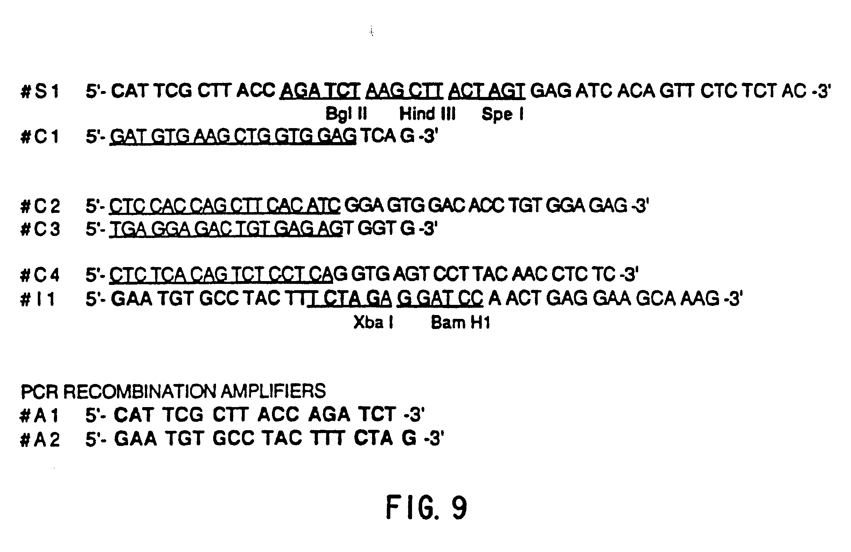

The chimaeric heavy chain portion of the recombinant antibody is derived from the murine 1B4 heavy chain variable region fused to the human constant region of a gamma 4 subtype obtained from a lambda library constructed by Flanagan and Rabbits, Nature 300: 709-713 (1982). The variable region of the chimaeric heavy chain is constructed from three DNA fragments representing a signal sequence, a portion of the murine heavy chain variable region, and an intronic sequence (Figure 8). Oligodeoxynucleotide primer pairs (Figure 9) are synthesized representing the primers necessary to generate by PCR amplification these three DNA fragments from about 10 ng of plasmid DNA templates obtained from M13VHPCR1 (Orlandi et al., Proc. Natl. Acad. Sci. USA 86: 3833-3837 [1989]) or the pSP72 intermediate vector containing the IgG2a heavy chain variable region previously used to determine the murine 1B4 CDR sequence. Amplification of the signal fragment, variable region fragment and intron-containing fragment was as described above. The agarose gel purified products are combined, about 10 ng of each product, with terminal oligodeoxynucleotide primer pairs (Figure 9) and the PCR-generated in vitro, recombined template is amplified using the standard procedures described above. Prior to subcloning into a BglII and BamHI digested intermediate vector pSP72 this recombined product is similarly digested and agarose gel purified. Individual clones are submitted to DNA sequence determination using Sequenase® and T7 and SP6 specific sequencing primers and one is chosen (p8950) for subsequent expression.

The gamma 4 heavy chain constant region is subcloned as about a 6.7 Kb HindIII fragment derived from the plasmid pAT84 (Flanagan and Rabbitts, Nature 300: 709-713 [1982]) into the Hind III site of the intermediate vector pSP72. This plasmid is then used as the template DNA from which a shortened version of the gamma 4 constant region is subcloned using PCR amplification and the primer pairs indicated in Figure 7. Eukaryotic expression vectors are constructed as described below. Expression vectors are defined herein as DNA sequences that are required for the transcription of cloned copies of genes and the translation of their mRNAs in an appropriate host. Such vectors can be used to express eukaryotic genes in a variety of hosts such as bacteria, blue-green algae, plant cells, yeast cells, insect cells and animal cells. The immunoglobulins may also be expressed in a number of virus systems. Specifically designed vectors allow the shuttling of DNA between hosts such as bacteria-yeast or bacteria-animal cells. An appropriately constructed expression vector should contain: an origin of replication for autonomous replication in host cells, selectable markers, a limited number of useful restriction enzyme sites, a potential for high copy number, and strong promoters. A promoter is defined as a DNA sequence that directs RNA polymerase to bind to DNA and initiate RNA synthesis. A strong promoter is one which causes mRNAs to be initiated at high frequency. Expression vectors may include, but are not limited to, cloning vectors, modified cloning vectors, specifically designed plasmids or viruses. The heavy chain immunoglobulin molecule is transribed from a plasmid carrying the neomycin (G418) resistance marker while the light chain immunoglobulin is transcribed from a plasmid carrying the hygromycin B resistance marker. With the exception of the drug resistance portion of these plasmids they are identical.

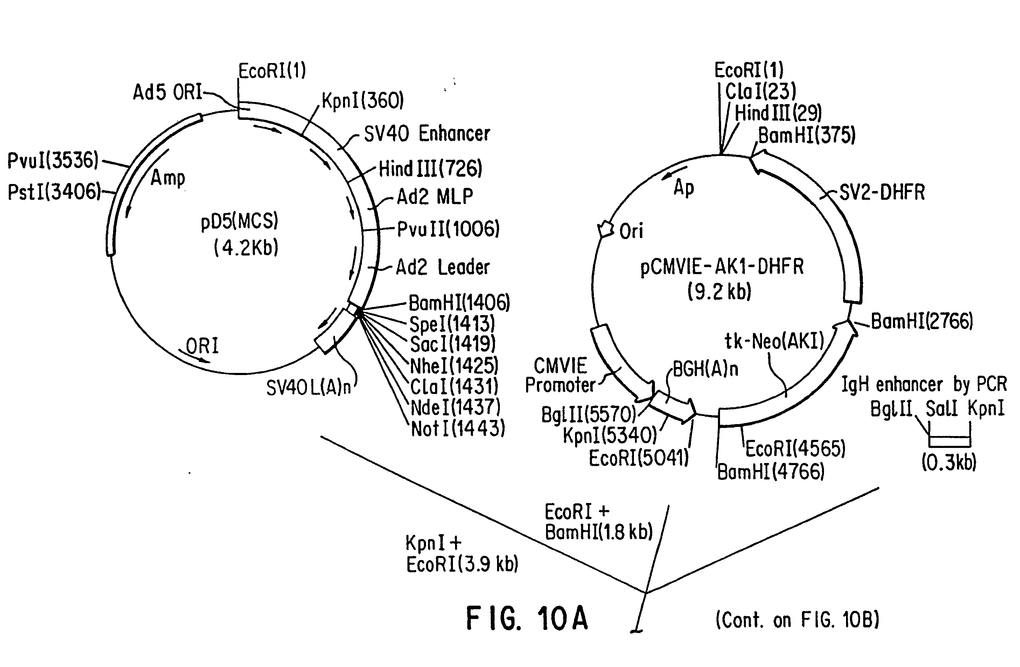

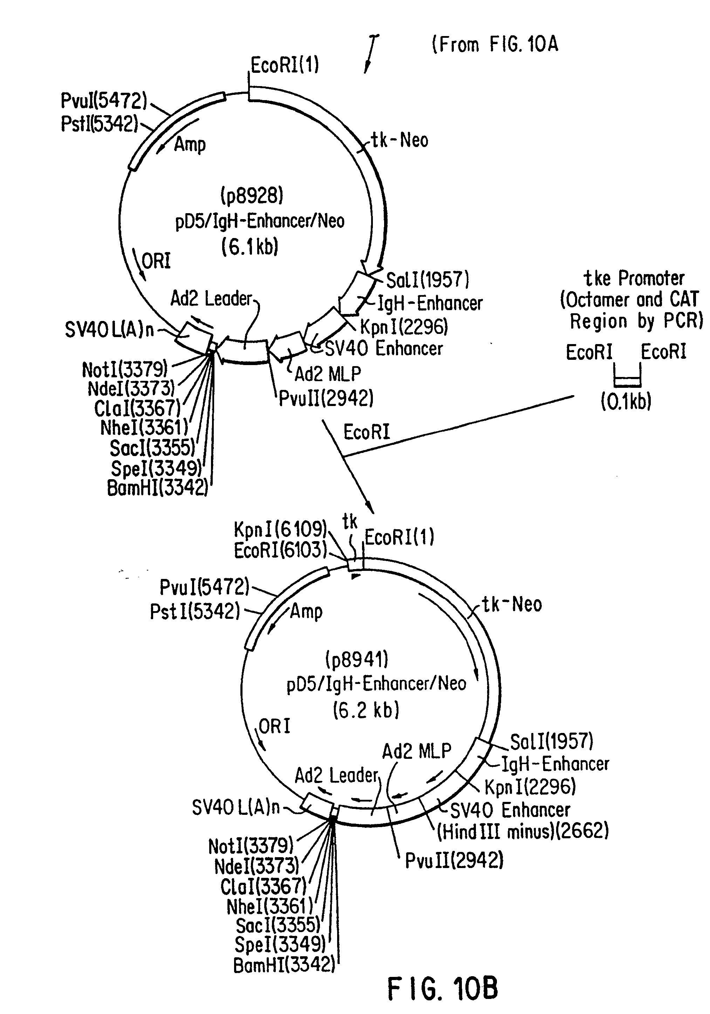

The preferred progenitor of the immunoglobulin expression vectors is the pD5 (Berkner and Sharp, Nucl. Acids Res. 13: 841-857 [1985]) eukaryotic expression vector which contains the origin of adenovirus replication, the SV40 enhancer domain, the adenovirus major late promoter, the adenovirus 2 tripartite leader, a 5′ splice donor from the adenovirus third leader and a 3′ splice acceptor derived from an immunoglobulin locus, a multiple cloning site placed in the Bam H1 site subsequent to receipt of the vector, and the SV40 late polyadenylation signal (Figure 10). The origin of replication is removed by digestion with Eco R1 and KpnI and replaced by two fragments representing the neo selectable marker gene (derived from plasmid pCMVIE-AK1-DHFR as an Eco R1/Bam H1 about 1.8 Kb fragment) and the Ig heavy chain enhancer (obtained as a PCR amplified fragment using human DNA as the template, and the oligodeoxynucleotides listed in Figure 7 as the primer pair, following its digestion with Bgl II and Kpn I). The resultant expression vector is found to lack a small portion of the TK promoter responsible for the transcription of the neomycin gene. This is replaced by insertion into the EcoRI site of about a 0. 14kb PCR amplified fragment derived from the CMVIE-AK1-DHFR DNA using the primer pair listed in Figure 7. The resultant heavy chain expression vector (p8941) is modified by removal of the indicated HindIII and XbaI sites using standard procedures. To convert this vector into one expressing the hygromycin B selectable marker the neomycin-resistance cassette is removed by digestion first with Eco R1 followed by DNA polymerase-directed fill in of the 5′ overhang, then subsequent SalI digestion. The about 1.9 kb hygromycin B expression cassett, TK promoter and TK polyadenylation signal flanking the hygromycin B gene, (obtained as a 1.8 kb BamH1 fragment in plasmid pL690, Gritz and Davies, Gene 25: 179-188[1981]) is removed from the plasmid pAL-2 by Bam H1 digestion and subcloned into the BamH1 site of the intermediate vector pSP72. The hygromycin B cassette is removed from this vector by digestion with SmaI and SalI and cloned into the expression vector linearized as described above to create a blunt end and SalI end DNA fragment.

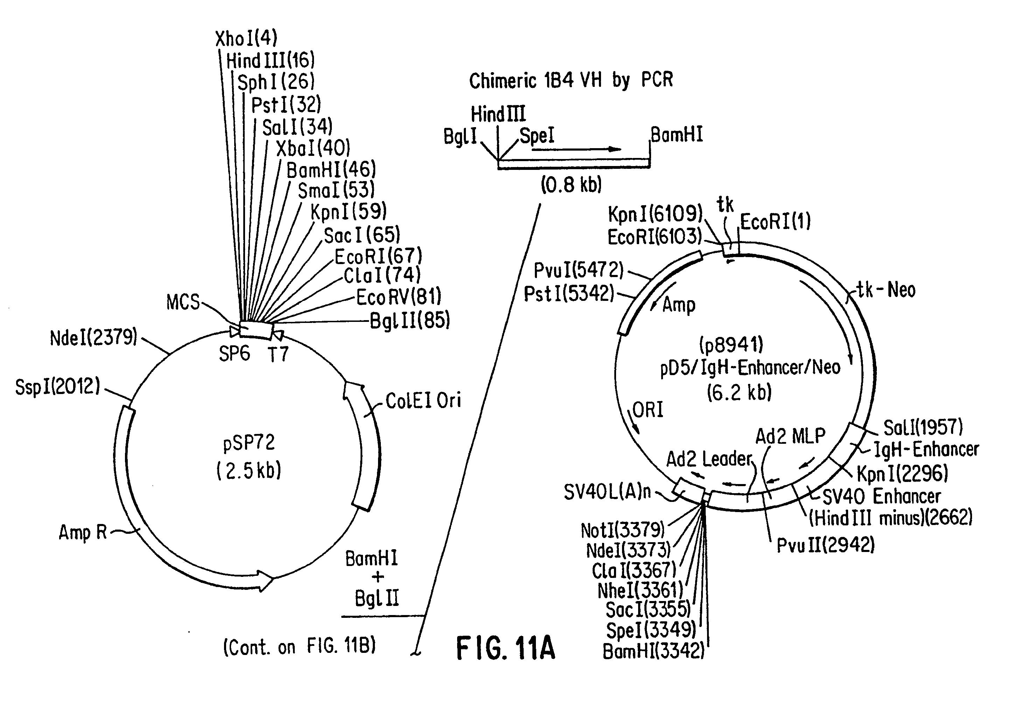

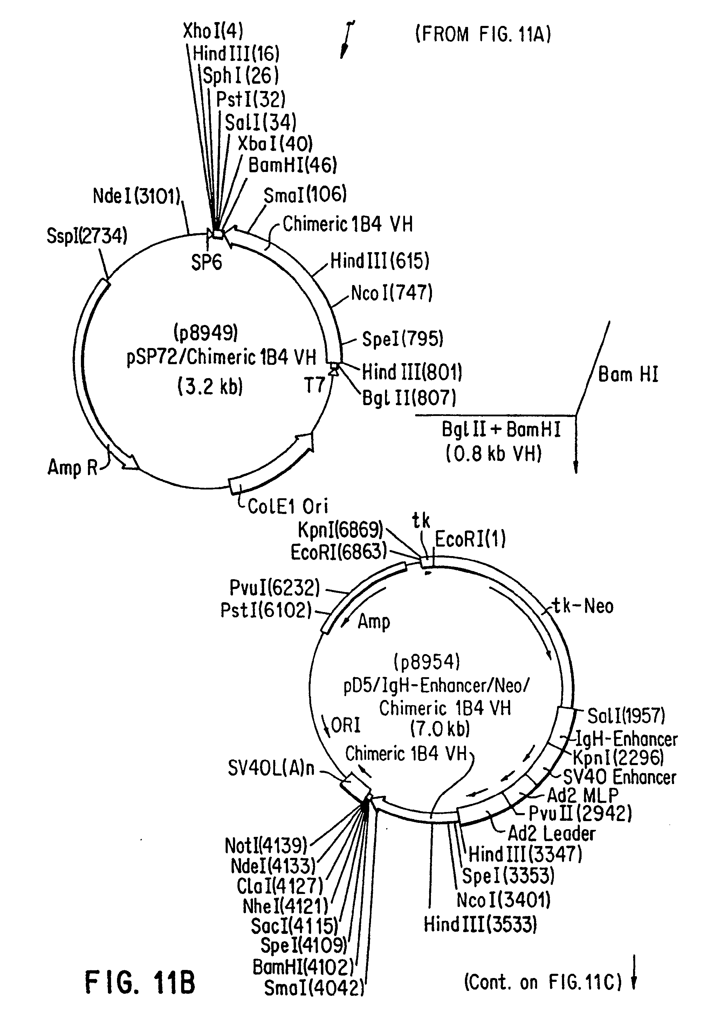

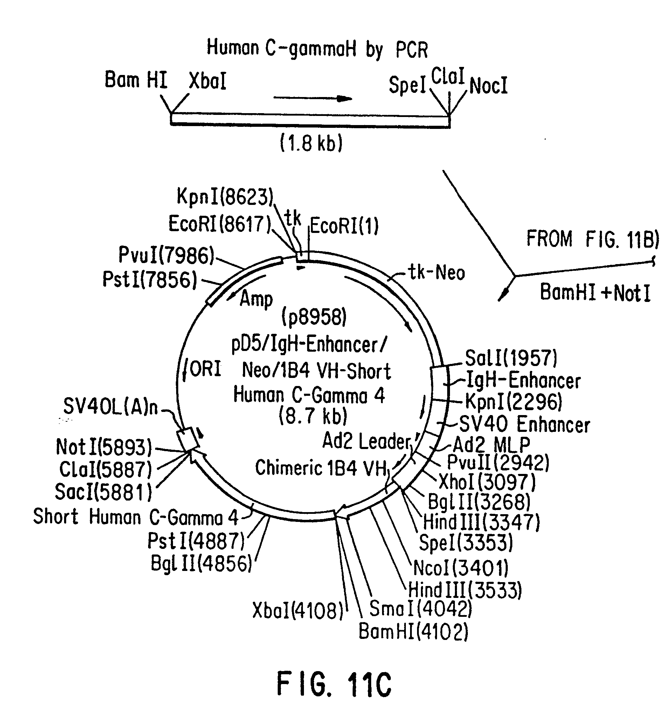

Expression of the 1B4 CDR-grafted kappa light chain is accomplished by transferring this cistron from the pSP72-based intermediate cloning vector (p8952) to the hygromycin B selectable eukaryotic expression vector (see Figure 6). An about 1.5 kb DNA fragment resulting from the endonuclease digestion of p8952 with Spe I and Cla I is purified by agarose gel electrophoresis and ligated into the expression vector which has previously been linearized, following digestion with the same two restriction enzymes, and agarose gel purified. The heavy chain eukaryotic expression vector is constructed in two steps (see Figure 11). First, the p8950 vector containing the modified heavy chain variable region of murine 1B4 Kb fragment is digested with Bgl II and Bam H1. The agarose gel purified 0.75 kb fragment is ligated into the BamH1 site of the p8941 vector and recombinant clones containing this fragment in the proper orientation are identified. Plasmid DNA from one such clone is linearized by Bam H1 digestion and ligated with a 1.78 Kb BamH1 fragment representing a short version of the human gamma 4 constant region, derived from plasmid pAT84 by PCR amplification. Following the identification of clones containing these inserts in the appropriate orientation, plasmid DNAs (one which is referred to as p8953) are grown and purified for transfection into recipient mammalian cells. Host cells for the expression of humanized monoclonal antibodies include, but are not limited to, human cells such as 293 cells, monkey cells such as COS-7 and CV-1P, and other mammalian cells such as CHO and NS0.

Equal amounts, about 10 µg, of the plasmids encoding the chimeric IgG4 heavy chain and the 1B4 CDR-grafted kappa light chain are transfected by standard calcium phosphate precipitation procedures into human 293 cells, and the monkey cells COS-7 and CV-1P. The culture supernants are assayed by a trapping Elisa (described below) for the secretion of human IgG4/kappa immunoglobulin. This Elisa assay is also employed for the quantitation of the amounts of a humanized 1B4 recombinant antibody expressed in conditioned mammalian cell growth medium.

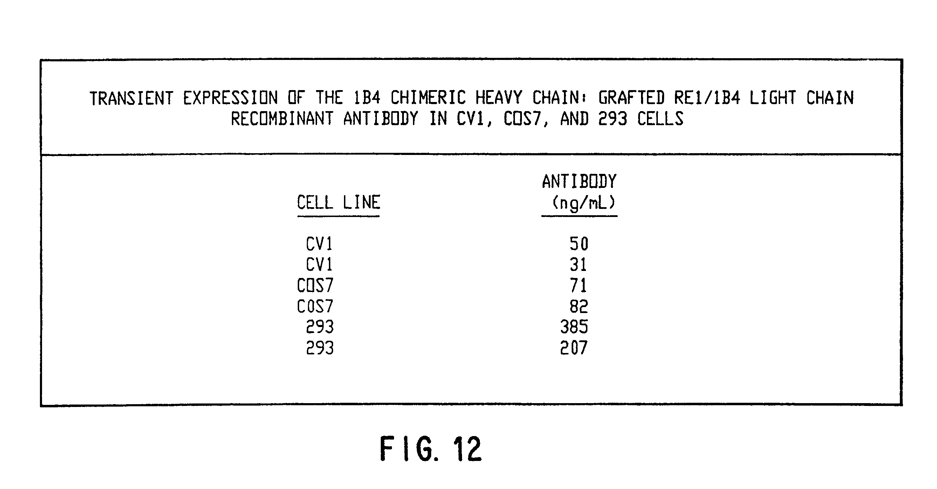

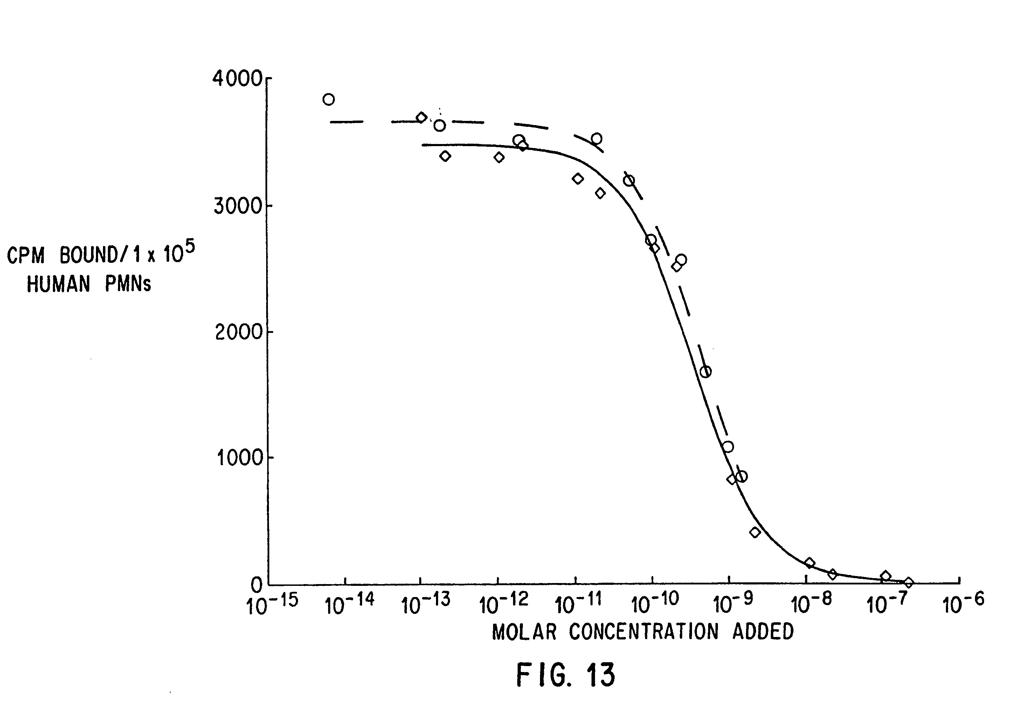

Immulon-2 (Dynatech Labs.) 96-well plates are coated overnight with about a 5 µg/ml solution of mouse anti-human kappa chain constant domain monoclonal antibody (cat. #MC009, The Binding Site, Inc., San Diego, CA) in about 0.1 M NaHCO₃ buffer (pH 8.2) at about 4°C, and blocked with about 1% bovine serum (BSA) in about 0.1M NaHCO₃ for about 1h at about 25° C. After this and all subsequent steps, washing was performed with phosphate buffered saline (PBS). The wells are then inoculated with conditioned medium containing recombinant anti-CD18 antibody, or with predetermined quantities of human IgG4/kappa purified by protein A Sepharose (Pharmacia Fine Chemicals) chromatography from human IgG4 myeloma serum (cat. # BP026,The Binding Site, Inc.) All samples are diluted in PBS containing about 0.05% Tween-20. About 100 µl aliquots are incubated for about 1h at about 37°C in triplicate, and standard calibration curves are constructed using IgG4 concentrations ranging from about 10 ng/ml to about 100 ng/ml. Bound and fully assembled human IgG4 (either native or recombinant 1B4-human IgG4 constructs) are detected with about 100 µl aliquots of a 1:500 dilution of mouse anti-human IgG4 Fc monoclonal antibody conjugated to alkaline phosphatase (cat #05―3822, Zymed Laboratories, Inc.) in phosphate buffered saline (PBS) containing about 1% BSA. After incubation for about 1h at about 37°C and subsequent washing, the quantities of bound conjugate are detected by incubating all samples with a 1 mg/ml solution of p-nitrophenyl phosphate in 0.1 M 2,2′amino-methyl-propanediol buffer, pH 10.3, for about 30 min at about 25°C. The adsorbance of the wells is determined with a UV Max ELISA plate reader (Molecular Devices) set at 405 nm. All supernatant fluids from the transfected cells are found to contain this immunoglobulin, though in various amounts (Figure 12). The antibody secreted by the transfected 293 cells is isolated by protein A chromatography and the the concentration of recombinant human anti-CD18 antibodies determined by the trapping Elisa described above, are used to compete with the binding of radiolabeled murine 1B4 to the CD18 ligand on the surface of activated human PMNs. Affinities of various recombinant human anti-CD18 (r-h-anti-CD18) antibody constructs are determined using a competitive ¹²⁵I-1B4 soluble binding assay with stimulated human polymorphonuclear leukocytes (PMNs). Purified murine anti-CD18 monoclonal antibody (50 ug) is iodinated using chloramine-T (Hunter and Greenwood, Nature 194: 495-496, 1962), and the radiolabeled antibody purified using a Bio-Sil TSK250 (Biorad) gel filtration HPLC column (which fractionates proteins in the range of 1-300 x 10³ daltons) equilibrated in 0.1 M phosphate buffer, pH 7.0. Effluent radioactivity is monitored with an in-line detector (Beckman Model 170; Beckman) and total protein measured at OD₂₈₀ with a Kratos Spectroflow 757 detector (Kratos). A single ¹²⁵I-1B4 peak composed of coincident OD₂₈₀ and radioactivity tracings characteristically elutes about 6 minutes, 30 seconds following sample injection. Specific activity of the product is generally about 10 µCi/µg protein, and 97-99% of the counts are precipitable with 10% trichloroacetic acid. The binding of this radiolabeled antibody is assessed on human PMNs purified on a discontinuous Ficoll/Hypaque gradient (English and Anderson, J. Immunol. Methods 5: 249-255, 1974) and activated with about 100 ng/ml phorbol myristate acetate for about 20 minutes at about 37°C (Lo et al., J. Exp. Med. 169: 1779-1793, 1989). To determine the avidity of antibodies for CD18 molecules on the PMN surface, about 1 x 10⁵ activated PMNs are incubated in a buffer such as Hanks balanced salt solution containing about 20 mM Hepes (pH 7.2), about 0.14 units aprotinin (Sigma Chemical Co.) and about 2% human serum albumin (binding buffer) containing about 1.3 ng ¹²⁵I-1B4 (2.8 x 10⁻¹¹ M) in the presence of increasing concentrations of unlabeled 1B4 antibody (about 10⁻⁷ to 10⁻¹⁵ M) in about a 300 µl reaction volume for about 1 h at about 4°C with constant agitation. Cell bound 1B4 is separated from the unbound antibody by centrifugation through a 0.5M sucrose cushion (4,800 x g, 3 minutes); the tubes are frozen on dry ice, and the tips cut off and counted with an LKB gamma counter. The IC₅₀ of the anti-CD18 antibody for the inhibition of ¹²⁵I-1B4 antibody binding is calculated using a four parameter fitter program (Rodbard et al., In, "Radioimmunoassay and Related Procedures in Medicine", International Atomic Energy Agency, Vienna, vol I, 469 - 504, 1978). The affinity of the various recombinant humanized anti-CD18 (r-h-anti-CD18) antibodies for the CD18 ligand is determined in a similar manner using murine ¹²⁵I-1B4 antibody and increasing quantities, as determined by the trapping Elisa, of unlabeled r-h-anti-CD18. The results of the binding assays are shown in Figure 13 and indicate that the avidity of the chimaeric heavy chain/grafted light chain recombinant 1B4 antibody is approximately that of the murine 1B4 monoclonal antibody.

The results described above show that an antibody with human isotype may be recombinantly expressed following the transfer of the antigen binding domains from a first animal (murine) light chain framework to a second animal (human) light chain framework one fused with a human kappa constant region, when combined with a chimaeric heavy chain (murine heavy chain variable region fused to a human gamma 4 constant domain) without loss in avidity for the antigen. It can be inferred from this result that the human REI light chain framework region does not alter the presentation of the murine 1B4 light chain CDRs and/or the contribution of the light chain CDRs to the antibody's avidity is minimal. Many of the examples of construction of recombinant human antibodies containing complementarity regions replaced by those found within murine monoclonal antibodies have resulted in loss of avidity for the ligand or antigen. Thus, although these transmutations are possible, the successful maintenance of avidity is not assured. The procedures described below demonstrate that when strict attention is payed to the framework regions, CDR domains may be transferred to those frameworks without the loss of avidity which accompanies their transfer to the "generic" frameworks employed by Winter, European Patent Publication No. 239,400, published September 30, 1987.

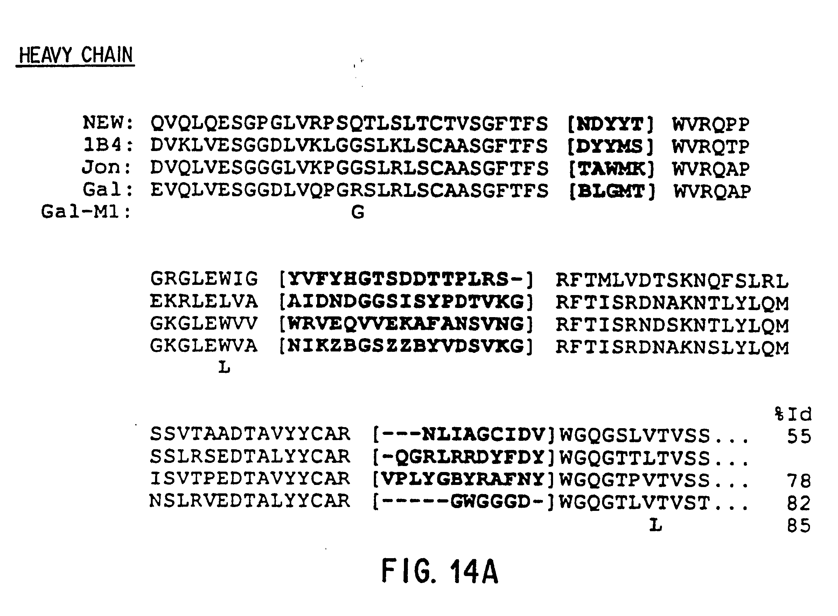

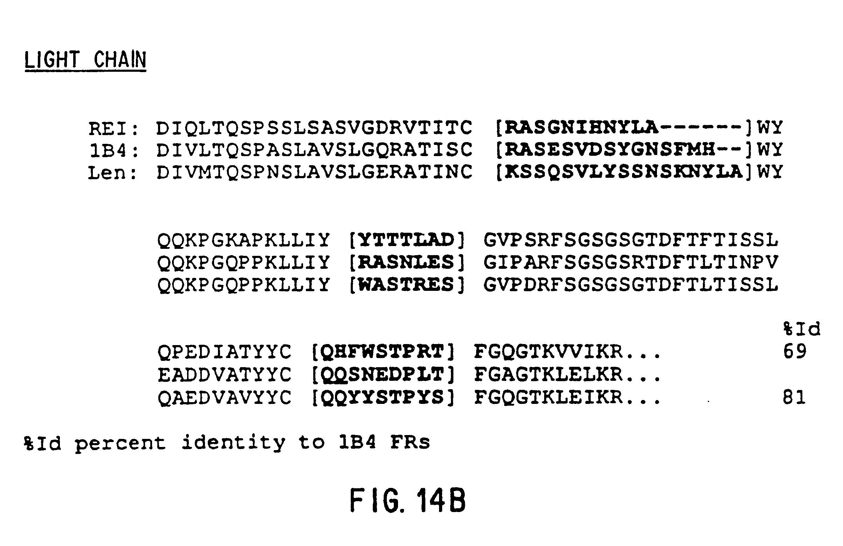

To identify human framework sequences compatible with the CDRs of, say, murine 1B4, human frameworks with a high degree of sequence similarity to those of murine 1B4 were identified. Sequence similarity was measured using identical residues as well as evolutionarily conservative amino acid substitutions. Similarity searches were performed using the murine 1B4 framework sequence from which the CDR sequences had been removed. This sequence was used to query a database of human immunoglobin sequences that had been derived from multiple sources. Sequences with a high degree of sequence similarity were examined individually for their potential as humanizing framework sequences. Special attention must be given to those framework residues which are not located or exposed on the surface of the antibody since these residues will play a critical role in the packing of the CDR supporting scaffolding. In this way, the human homologue providing the murine CDRs with the structure most similar to their native murine framework was selected for subsequent construction of the humanized variable region (see Figure 14). It should be noted that in the present invention the heavy and light chain framework sequences chosen for grafting need not be derived from the same human antibody. That is to say, using the above mentioned criteria for choosing human frameworks the entire accumulated human nucleic acid and protein databases may be searched for the desired matching sequences. The ideal light chain framework may come from one immunoglobulin sequence while the heavy chain framework may come from another. Should human frameworks of sufficient similarity not be identifiable from compiled sequences, it is possible to isolate from human genomic DNA a group of closely related variable regions using recombinant technology. Thus, a degenerate 5′ upstream oligodeoxynucleotide primer may be designed from the conserved sequences within the amino-terminus of each of the various human FR1 regions and paired with a degenerate 3′ downstream oligodeoxynucleotide primer fashioned from the FR3 sequence determined from the murine monoclonal whose CDRs one wishes to transfer into a human context. These primer pairs are then used to PCR amplify from a human genomic template those DNA sequences which are flanked by the primer pair. The resulting DNAs may then be cloned and the DNA sequence derived from individual members will describe various murine-related human variable regions. The paucity of somatic mutations in framework residues and the conservation of amino acid sequence between mouse and man make this approach possible.

The construction of a complete recombinant human IgG4 antibody, whose heavy and light chain variable domains contain the CDR residues of the murine monoclonal antibody, with complete retention of the specificity and avidity of the parent murine monoclonal antibody is disclosed. The construction of the CDR-grafted light chain framework derived from the human sequence of REI fused with a human kappa light chain constant region is described above.

The murine variable region framework sequence, devoid of CDR sequences, is used to query a database of complete human variable region sequences. The human sequences that are most similar to the murine framework region are then analyzed individually to determine both their sequence identity and similarity to the murine framework region. In the case of murine 1B4 these sequences include, but are not limited to, Gal and Jon, chosen because of their high degrees of both similarity and identity with the murine 1B4 heavy chain sequence. The Gal FR has been shown to be 85% similar and 79% identical to murine 1B4, while the Jon FR has been shown to be 88% similar and 75% identical to 1B4. These values are based upon the Dayhoff similarity matrix of evolutionarily conserved amino acid substitutions (R. M. Schwartz, M. O. Dayhoff, in *Atlas of Protein sequence and structure* M. O. Dayhoff, Eds. (National Biomedical Research Foundation, Washington, DC [1979]) (see Figure 14). To prepare a recombinant DNA encoding the murine heavy chain CDRs in the context of each of these frameworks the following procedures are performed.

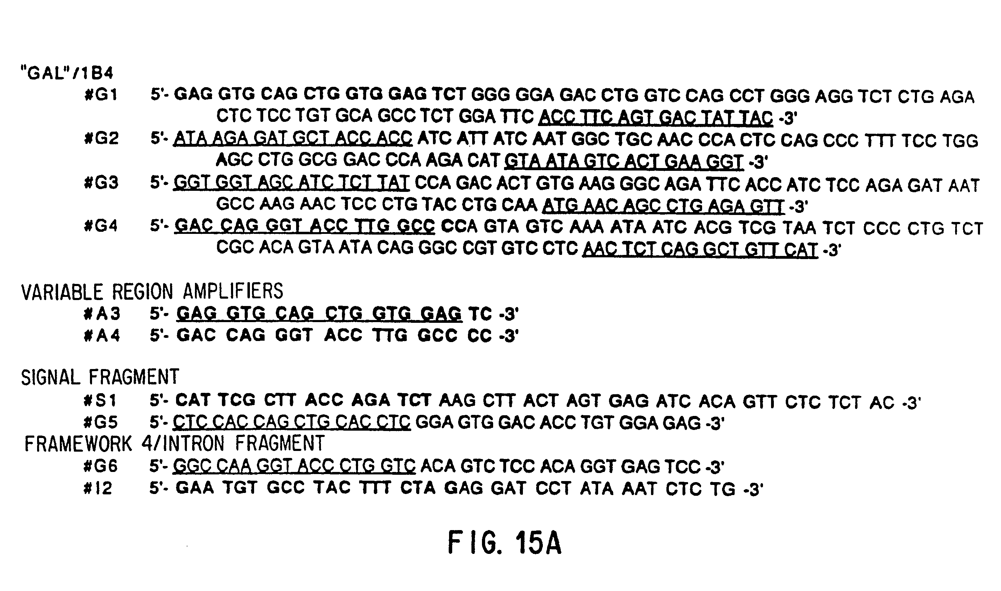

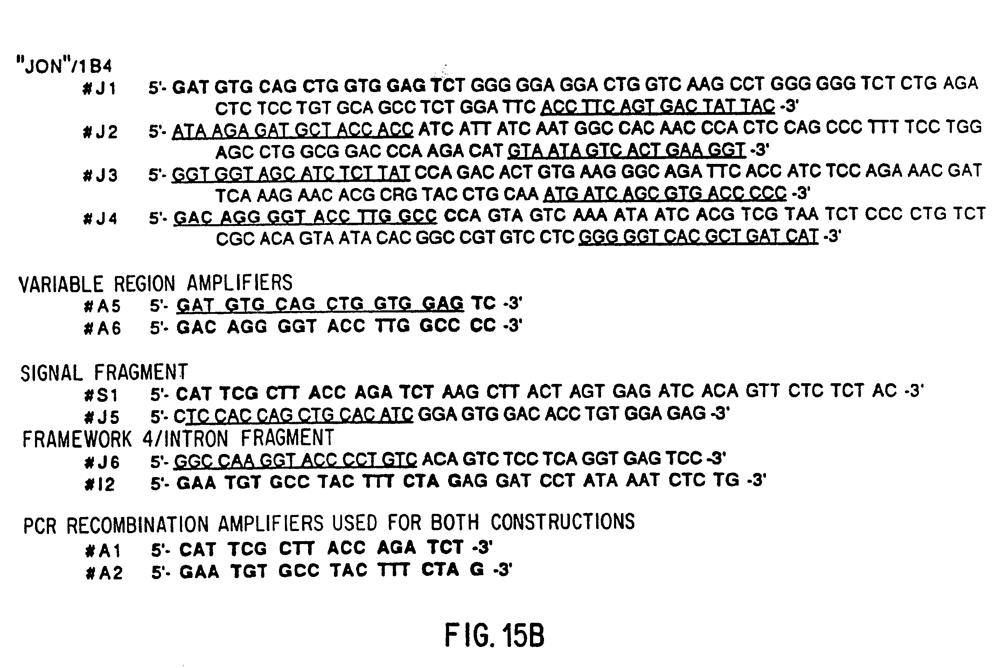

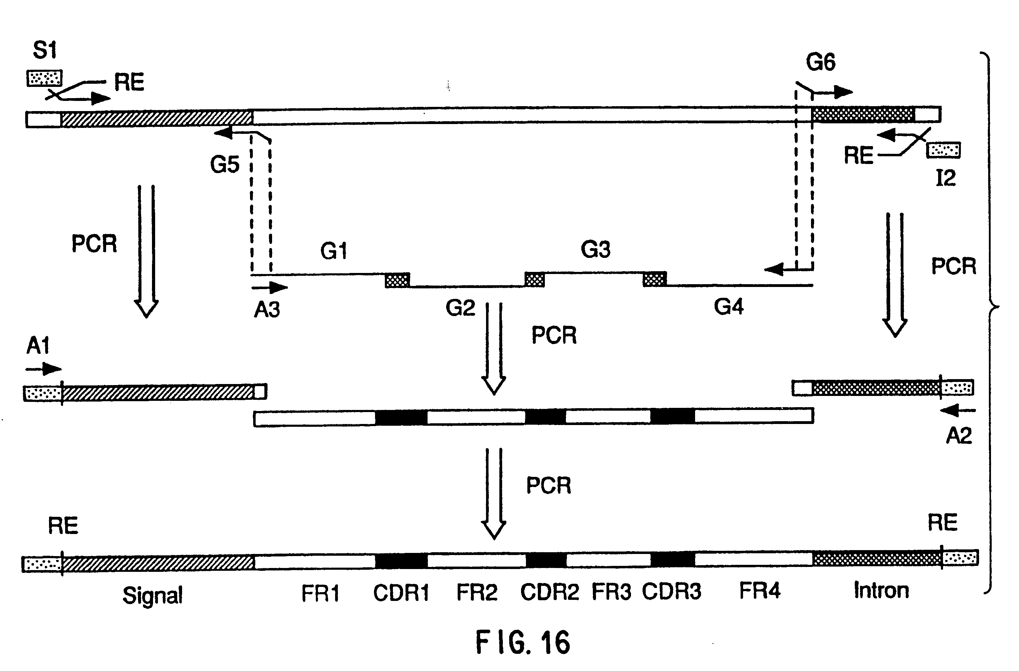

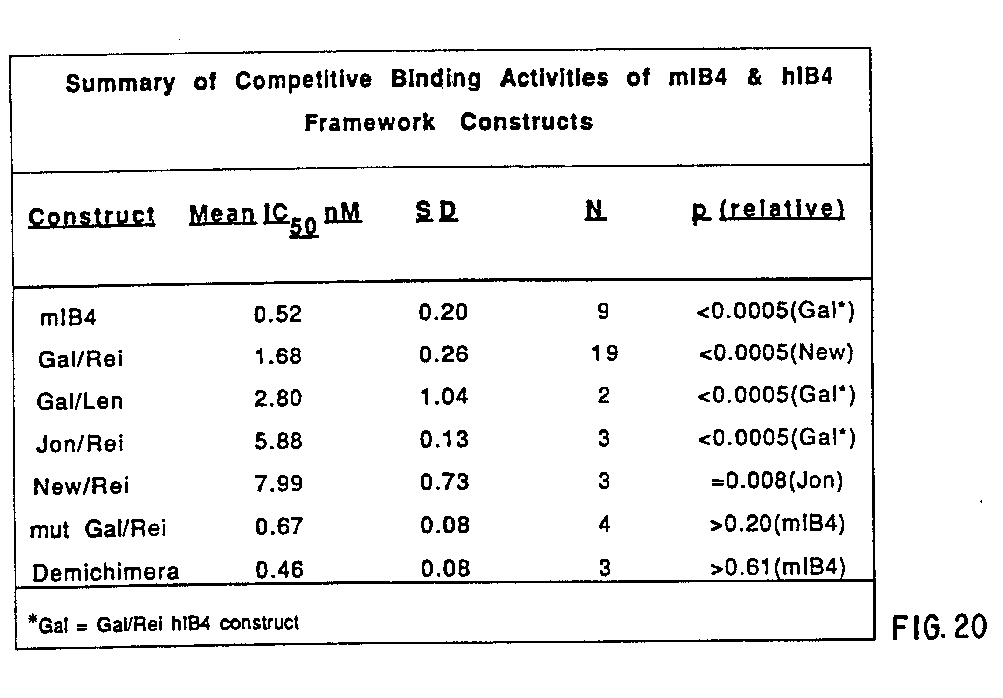

Two sets of four long oligodeoxynucleotides are synthesized. When each set is combined, they encode the 1B4 heavy chain CDRs and the chosen human heary chain variable region framework. The four oligodeoxynucleotides of a set, about 1 pmole of each, are combined in a PCR reaction with Taq polymerase and about 50 pmoles of each terminal amplifying oligodeoxynucleotide (Figure 15, Figure 16). By virtue of the complementary ends of the single-stranded oligodeoxynucleotides, the polymerization-denaturation-polymerization cycles of the polymerase chain reaction result in the formation, and subsequent amplification, of the combined sequences. Following about 25 cycles of amplification the combined 0.4 Kb fragment is electrophoretically purified from an agarose gel. In parallel, two DNA fragments representing amino terminal sequences encoding the signal peptide and carboxy terminal sequences encoding framework 4, splice donor, and intronic sequences are amplified using oligodeoxynucleotide primer pairs (Figure 15) and the NEWM containing plasmid DNA template M13VHPCR1 (described above). These two fragments are agarose gel purified, as above, and about 10 ng of each is combined with about 10 ng of the amplified grafted variable region fragment, Taq polymerase, about 50 pmoles of each of the terminal primers (Figure 15) and the mixture was PCR amplified. The resultant 0.85 Kb fragment is digested with restriction enzymes Spe I and BamH1. Following agarose gel electrophoresis, the purified DNA fragment is ligated into the heavy chain expression vector, p8958 (see Figure 11), in place of the chimaeric variable region. In this way, two unique heavy chain frameworks containing the grafted murine CDRs (Jon/1B4 and Gal/1B4) are constructed. Each fully grafted heavy chain expression vector plasmid is co-transfected with the fully grafted REI/1B4 light chain expression vector plasmid into 293 cells and the recombinant human antibody is present in conditioned medium. The Gal/1B4:REI/1B4 heterodimeric human (fully humanized) recombinant antibody is isolated by protein A chromatography. The avidity of this antibody for the CD18 ligand displayed on the surface of activated human PMNs is compared with that of the chimaeric/grafted antibody, described above, and the 1B4 murine monoclonal antibody parent. Figure 20 shows that although each hetero-dimeric antibody contains the same set of six CDRs, they do not exhibit identical avidity for the ligand. Thus, the avidity of an antibody molecule relies upon the variable region framework structure in which the CDRs are presented. The parent murine monoclonal antibody demonstrates an IC₅₀ of about 0.5 nM while the Gal/Rei heterodimer has an IC₅₀ of about 1.6 nM.

To determine the relative contribution of the heavy and light chain variable regions to the enhanced avidity of the Gal/REI grafted hetero-dimer, second light chain and heavy chain frameworks were constructed containing the 1B4 CDR sequences. These frameworks, termed Len and mutant Gal or Gal-M1 were chosen from the human immunoglobulin database by virtue of their high degree of similarity to the light chain FR and heavy chain FR of murine 1B4 (Figure 14). The Len FR shows a similarity of 90% and an identity of 81% when compared to murine 1B4. The resulting recombinant antibodies which specifically bind to CD18 antigen or receptor are termed recombinant human anti-CD18 antibodies (r-h-anti-CD18 Abs).

This invention further relates to a method of inhibiting the influx or migration of leukocytes capable of expressing CD18 antigen (leukocyte integrin, beta-2 subunit) on their surface into a site of inflammation or a tissue area or organ that will become inflamed following an influx of the cells. The inflammation which is the target of the method of the present invention may result from an infection with pathogenic microorganisms such as gram-positive and gram-negative bacteria, parasites and fungi. The response may also be induced by viruses and non-infectious means such as trauma or reperfusion following myocardial infarction or stroke, immune responses to foreign antigen and autoimmune responses.

The recombinant human anti-CD18 antibodies are useful in the treatment of inflammation in lung, central nervous system, kidney, joints, endocardium, pericardium, eyes, ears, skin, gastrointestinal tract and urogenital system. Disease states in which the recombinant human anti-CD18 antibodies are useful as therapeutic agents include, but are not limited to: infectious diseases where active infection exists at any body site, such as meningitis; conditions such as chronic or acute secondary inflammations caused by antigen deposition; and other conditions such as, encephalitis; arthritis; uveitis; colitis; glomerulonephritis; dermatitis; psoriasis; and respiratory distress syndrome associated with sepsis and/or trama. Other inflammatory diseases which may be responsive to recombinant human anti-CD18 antibody include, but are not limited to, immune disorders and conditions involving T-cell and/or macrophage attachment/recognition, such as acute and delayed hypersensitivity, graft vs. host disease; primary auto-immune conditions such as pernicious anemia; infection related auto-immune conditions such as Type I diabetes mellitis; flares during rheumatoid arthritis; diseases that involve leukocyte diapedesis, such as multiple sclerosis; antigen-antibody complex mediated diseases including certain of the secondary infection states listed above; immunosuppression; and transplant rejection. Inflammatory conditions due to toxic shock or trauma such as adult respiratory distress syndrome and reperfusion injury; and disease states due to leukocyte dyscrasias and metastasis, are included within the scope of this invention.

The present invention is also applicable to the inhibition of leukocyte-endothelial attachment for diagnostic and therapeutic purposes; such as the iatrogenic opening of the endothelium to prevent the ingress of leukocytes during the ingress of a dye or image enhancer into tissue, or to allow the selective entry of a therapeutic drug in the instance of chemotherapy; or to enhance the harvesting of leukocytes from patients.

Recombinant human anti-CD18 antibodies or an active fragment thereof can be used to treat the above mentioned diseases. An active fragment will include the F(ab′)2, the Fab and any other fragment that can bind to the CD18 antigen. Recombinant human anti-CD18 antibodies can be administered alone for non-infectious disease states or combined with antibiotics or other anti-infective agents for the treatment of infectious diseases for reasons di scussed above. Administration will generally include the antibodies and other substance in a physiologically acceptable medium or pharmaceutical carrier. Such physiologically acceptable media or pharmaceutical carriers include, but are not limited to, physiological saline, phosphate buffered saline, phosphate buffered saline glucose, buffered saline and the like. The antibodies and any anti-infective agent will be administered by parenteral routes which include intravenous, intramuscular, subcutaneous and intraperitoneal injection or delivery.

The amount of the antibodies and the mixture in the dosage form is dependent upon the particular disease state being treated. The amount of the recombinant human anti-CD18 antibody utilized in a dosage form can range from about 1 to about 1,000 mg, with a range of from about 10 mg to about 100 mg being preferred. The antibodies can be administered daily or less than daily as determined by the treating physician.

The following examples illustrate the present invention without, however, limiting the same thereto.

### EXAMPLE 1

### Preparation of a Grafted / Chimaeric Recombinant Antibody

An antibody was produced in which the variable domain of the light chain comprises the framework regions of a human light chain and the CDRs from a mouse light chain, while the variable domain of the heavy chain is derived entirely from the murine heavy chain. The light chain framework regions were derived from human myeloma protein REI (Orlandi, et al., Proc. Natl. Acad. Sci. USA 86: 3833-3837[1989]; Riechmann et al., Nature 332: 323-327[1988]; European Patnet Application, Publication No. 239,400) for which the crystallographic structure has been determined. The CDR sequences from the murine monoclonal antibody 1B4 which binds to CD18 (the beta subunit of the leukocyte integrin beta-2 family which includes: LFA-1, Mac-1, and p150.95) were derived as follows. The hybridoma designated 1B4 which produces 1B4 monoclonal antibody was deposited under the Budapest Treaty at the International Depository Authority: American Type Culture Collection, 12301 Parklawn Drive, Rockville, MD, 20852. Viability was determined on June 6, 1989 and the hybridoma was designated HB 10164. Previous experiments had determined this antibody to be an IgG 2a with a kappa light chain (Wright et al., Proc. Natl. Acal. Sci. USA 80: 5699-5703 [1983]).

Total RNA was extracted from the 1B4 myeloma cells using standard methods involving cellular solubilization with guanidinium isothiocyanate (Chirgwin et al., Biochem. 18: 5294-5299[1979]). Sets of degenerate oligonucleotide primers (Figure 1) representing sequences within framework 1 of the murine kappa light chain variable region and kappa light chain constant domain, or those within framework 1 of the murine IgG2a heavy chain variable region and heavy chain constant CH1 domain were synthesized by standard phosphoramidite procedures on an Applied Biosystem 381A DNA synthesizer. Removal of the oligodeoxynucleotides (oligos) from the resin was accomplished by treatment with concentrated NH₄OH followed by desalting on a NAP-5 column (Pharmacia) with H₂O elution (when the oligos were <45 bases in length), or by use of an OPC column (Applied Biosystems Inc) with 20% acetonitrile elution (when the oligos were >45 bases in length), as recommended by the manufacturers. Total RNA (2µg) was reversed transcribed for 30′ at 42°C using Moloney MLV reverse transcriptase (200 units, BRL) and 10 pmoles of the constant region complementary strand primers representing either heavy or light chain in a buffer (final volume of 20 µl) containing 50 mM Tris HCl, pH 8.3, 75 mM KCl, 3 mM MgCl₂, 10 mM DTT, and 20 units of RNAsin (Pharmacia). The reverse transcriptase was heat inactivated (95°C, 5′) and the reactions were made to contain in 100 µl of PCR buffer (10 mM Tris HCl, pH 8.3, 50 mM KCl, 1.5 mM MgCl₂, 0.01% gelatin, 200 µM each dNTP), 50 pmoles of each of the paired primers, and 2.5 units of Taq polymerase (Perkin Elmer/Cetus). Polymerase chain reaction (PCR) amplification was carried out essentially as described by Saiki et al., Science 230: 1350-1354 (1985) and others (Mullis et al., Cold Srping Harbor Symp. Quant. Biol. 51: 263-273[1986], Dawasaki and Wang, PCR Technology, Princples and Applications for DNA Amplification, Erlich, Ed., Stockton Press, NY, pp. 89-97[1989], Tung et al., ibid. pp. 99-104[1989]). Forty five cycles of amplification by a DNA Thermal Cycler (Perkin Elmer Cetus Instruments) (2′, 94°C; 2′, 55°C; 2′ 72°C) were followed by gel purification of the anticipated 400+ base pair (bp) DNA fragments (Figure 2). Prior to subcloning the DNAs into a blunt-ended intermediate plasmid (pSP72, Promega) they were terminally phosphorylated using T4 polynucleotide kinase (Boehringer Mannheim). Frozen competent E.coli were thawed on ice and 100 µl aliquots were distributed into wet ice chilled polypropylene tubes. DNA (1-10 ng) from the ligation mixture was dispensed with aggitation into these tubes and the mixture was incubated on ice was for 30 minutes. The E. coli cells were heat-shocked by incubation at 42° C for 45 seconds, then chilled for 2 minutes on ice. Room temperature S.O.C. (Hanahan, D., J.Mol. Biol. 166: 557[1983]) was added and the cultures were shaken at 225 RPM at 37° C for 60 minutes. Aliquots of the cultures were spread on LB agar plates containing
100 µg/mL ampicillin and these plates were incubated overnight at 37°C to allow for colony growth.

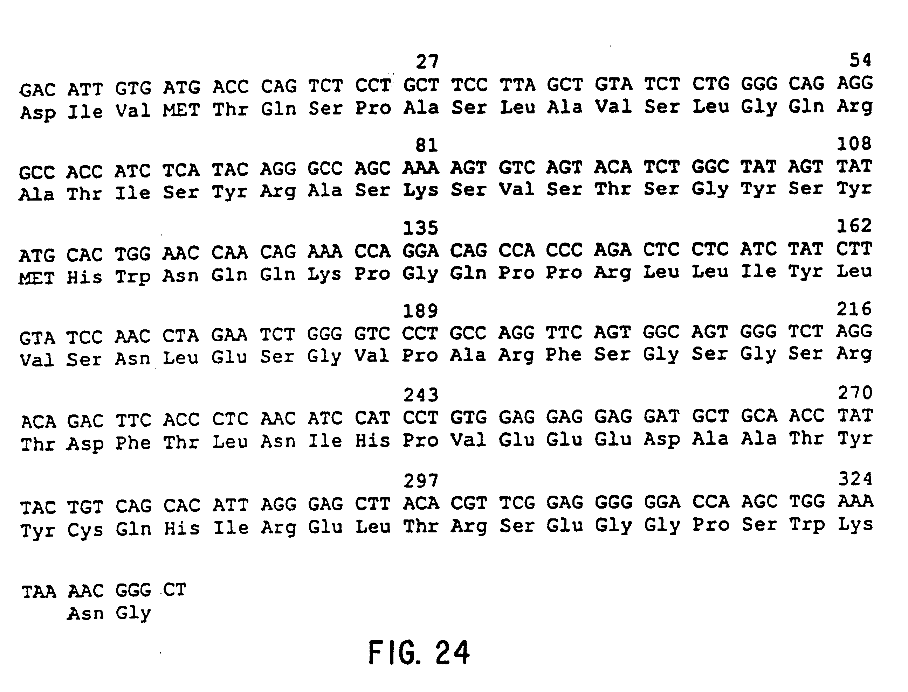

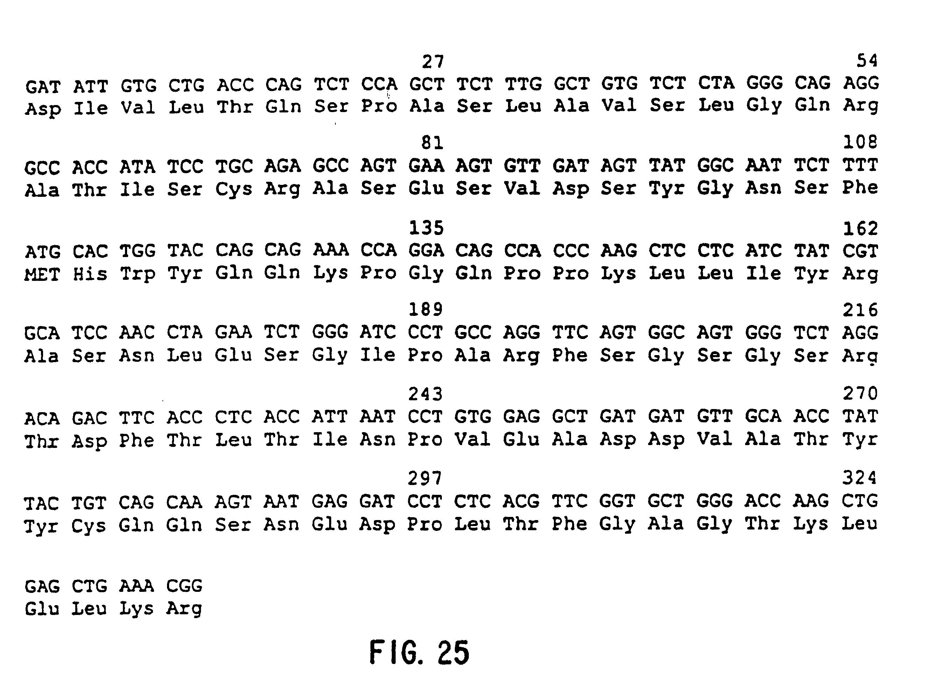

Multiple clones representing these PCR amplified sequences were isolated form DH5 transformed E.coli plated on LB agar plates containing 50 µg/ml ampicillin, grown by described procedures (Maniatis et al., Molecular Cloning, A Laboratory Manual, Cold Spring Harbor Laboratory, Cold Spring Harbor, NY, 1982), plasmid DNAs were extracted from the bacteria using the DNA preparation procedures of Birnboin and Doly Nucleic Acid Res. 7: 1515 (1979), and the double-stranded plasmid DNAs were submitted to DNA sequence determinations using Sequenase® (United States Biochemicals) and T7 and SP6 specific sequencing primers (Boehringer Mannheim) using the protocols recommended by the manufacturer. A unique DNA sequence representing a murine IgG2a heavy chain variable region was obtained, but two kappa light chain variable regions were represented within the cloned population (Figure 3). To distinguish which sequence belonged to the 1B4 MAb, the 1B4 MAb was reduced with DTT and purified light chains were subjected to N-terminal amino acid sequencing using the Applied Biosystems 477A sequencer. Although stretches of amino acid residues were identical to the mMAb 1B4 observed within the 1B4 light chain -1 sequence predicted from the cDNA, 1B4 light chain -2 (Figure 25) was deemed to be the actual sequence of the MAb 1B4 light chain. This is consistent with the determined DNA sequence of the light chain-1 molecule (Figure 24) which suggests it represents a murine kappa light chain variable region of subgroup III containing a mutation in the CDR3/FR4 region whose consequence is peptide chain termination.

Replacement of the human REI variable region CDRs with those unique to MAb 1B4 took place as follows. The REI framework (obtained as the RF form of the M13 vector M13VKPCR1, Orlandi et al., Proc. Natl. Acad. Sci. USA 86: 3833 (1989), with its signal peptide leader and intronic sequences, was subcloned into the intermediate vector pGEM3Z (Promega), as was the NEW or NEWM heavy chain variable region framework (obtained in the form of the M13 vector M13VHPCR1, Orlandi et al., supra). Eight oligodeoxynucleotides (Figure 4) were synthesized representing the primers necessary to generate by PCR amplification four DNA fragments. Incorporated into all but the terminal oligodeoxynucleotides were those sequences corresponding to the mMAb 1B4 light chain CDRs and at least 15 bases of 5′―terminal complementarity (see Figure 5). The appropriate primer pair (50 pmole each) was combined with 10 ng of REI framework-containing plasmid DNA, 2.5 units of Taq DNA polymerase, PCR reaction components and buffer, and thirty (30) cycles of PCR amplification ensued (cycle periods, as above). The products of the four reactions, purified by agarose gel electrophoresis, were combined (10 ng of each DNA fragment) along with a terminal oligodeoxynucleotide primer pair (amplifier) (Figure 4), Taq DNA polymerase, PCR reaction components and buffer, and the subsequent recombined fragments were amplified, as described above, for thirty cycles (see Figure 5). Following restriction endonuclease digestion with HindIII and XbaI the amplified DNA was purified from an agarose gel and subcloned into these same sites of an intermediate vector pSP72 (Promega) which contained the human kappa light chain constant region, obtained as follows. DNA (1µg) purified from a human B cell line (GM01018A; NIGMS Human Genetic Mutant Cell Repository, Institute for Medical Research, Camden, N.J. 08103) was used as a template for the oligodeoxynucleotide primers described in Figure 7 to PCR amplify a 920 base pair fragment containing the splice acceptor for the human kappa light chain constant domain, the exon and a portion of its 3′-untranslated region (PCR primer pair choice was selected based on the kappa constant region sequence described by Hieter et al., Cell 22: 197-207[1980]). The PCR product was purified by agarose gel electrophoresis, digested with BamH1 endonuclease, and subcloned into pSP72 (Promega) previously linearized with BamH1.

The individual clones (p8982) representing the pSP72 intermediate vector containing both the 1B4 grafted light chain variable region derived from REI and the human kappa constant region derived by PCR amplification of human DNA were used to verify the DNA sequence of the grafted light chain variable region. The chimaeric heavy chain portion of the recombinant antibody was derived from the murine 1B4 heavy chain variable region fused to the human constant region of gamma 4 subtype obtained from a lambda library constructed by Flanagan and Rabbitts, Nature 300: 709-713 (1982).

The variable region of the chimaeric heavy chain was constructed from three DNA fragments representing a signal sequence, a portion of the murine 1B4 heavy chain variable region, and an intronic sequence (Figure 8). Oligodeoxynucleotide primer pairs (Figure 9) were synthesized representing the primers necessary to generate by PCR amplification these three DNA fragments from 10 ng of plasmid DNA template containing either the NEW heavy chain variable region (M13VHPCR1) or a pSP72 intermediate vector containing the IgG 2a heavy chain region previously used to determine the murine 1B4 CDR sequence. Amplification of the 225 bp signal fragment, 350 bp variable region fragment, and 230 bp intron-containing fragment was performed as described above. The agarose gel purified products were combined (10 ng of each product) with terminal primer pairs (Figure 9) and the PCR-generated in vitro recombined template was amplified using the standard procedure described above for recombining the fragments comprising the 1B4 grafted REI light chain variable region. Prior to subcloning into a BglII and BamHI digested intermediate vector (pSP72) (Promega) this recombined product was similarly digested and agarose gel purified. DNA was obtained following growth of individual bacterial clones and submitted to DNA sequence determination using Sequenase® and T7 and SP6 specific sequencing primers in order to verify the sequence of the reconstructed variable region and its flanking domains.

The gamma 4 heavy chain constant region was subcloned as a 6.7 Kb HindIII fragment derived from the plasmid pAT84 (Flanagan and Rabbitts, supra) into the Hind III site of the intermediate vector pSP72 (Promega). This plasmid (p8947) was then used as the template DNA from which a shortened version of the gamma 4 constant region was obtained using the standard PCR amplification procedures described above and the primer pairs indicated in Figure 7. Eukaryotic expression vectors were constructed as described below such that the heavy chain immunoglobulin molecule was transribed from a plasmid carrying the neomycin (G418) (Rothstein and Reznikoff, Cell 23: 191-199[1981]) resistance marker, while the light chain immunoglobulin was transcribed from a plasmid carrying the hygromycin B resistance marker (Gritz and Davies, Gene 25: 179-188[1983]). With the exception of the drug resistance portion of these plasmids they are identical.

The progenitor of the immunoglobulin expression vectors was the pD5 eulcaryotic expression vector (Berkner and Sharp, Nucl. Acids Res. 13: 841-857[1985]) which contained the origin of adenovirus replication, the SV40 enhancer domain, the adenovirus major late promoter, the adenovirus 2 tripartite leader, a 5′ splice donor from the adenovirus third leader and a 3′ splice acceptor derived from an immunoglobulin locus, a multiple cloning site, and the SV40 late polyadenylation signal (Figure 10). The origin of replication was removed by digestion with Eco R1 and KpnI and replaced by two fragments representing the neo selectable marker gene (derived from plasmid pCMVIE-AK1-DHFR (Silberklang et al., Modern Approaches to Animal Cell Technology, Ed. Spier et al., Butterworth, U.K., [1987]) as an Eco R1/Bam H1 1.8 Kb fragment) and the Ig heavy chain enhancer (obtained as a PCR amplified fragment using standard procedures described above and human DNA as the template; the oligonucleotide primer pair is listed in Figure 7) following its digestion with Bgl II and Kpn I. The resultant expression vector was found to lack a small portion of the TK promoter responsible for the transcription of the neomycin gene. This was replaced by insertion into the EcoRI site of a 0.14 kb PCR amplified fragment derived from the CMVIE-AK1-DHFR DNA using the primer pair also listed in Figure 7. The resultant heavy chain expression vector was subsequently modified by removal of the indicated HindIII and XbaI sites. To convert this neomycin selectable vector (p8941) into one expressing the hygromycin B selectable marker (p8942) (Figure 10) the neomycin-resistance cassette was removed by digestion first with Eco R1 followed by DNA polymerase-directed fill in of the 5′ overhang, then subsequent SalI digestion. The 1.9 kb hygromycin B expression cassette [TK promoter and TK polyadenylation signal flanking the hygromycin B gene obtained from Gritz and Davies, Gene 25: 179-188 (1983), as the 1.9 kb BamH1 fragment in plasmid (pLG90)] was removed from the plasmid pAL-2 by Bam H1 digestion and subcloned into the BamH1 site of the intermediate vector pSP72 (Promega). The hygromycin B cassette was removed from this vector by digestion with SmaI and SalI and cloned into the expression vector linearized as described above to create a blunt end and SalI end DNA fragment.

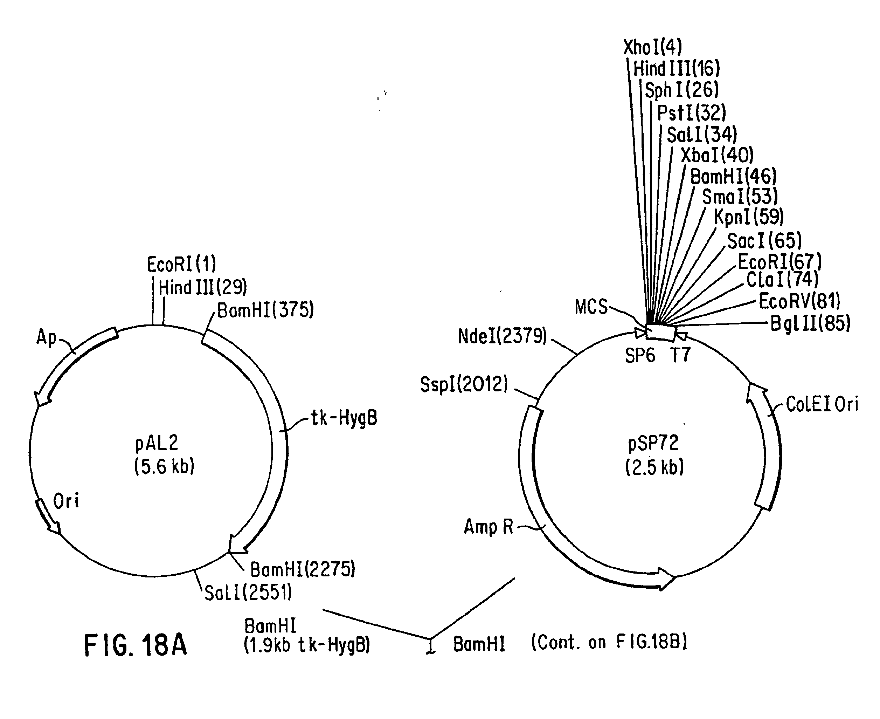

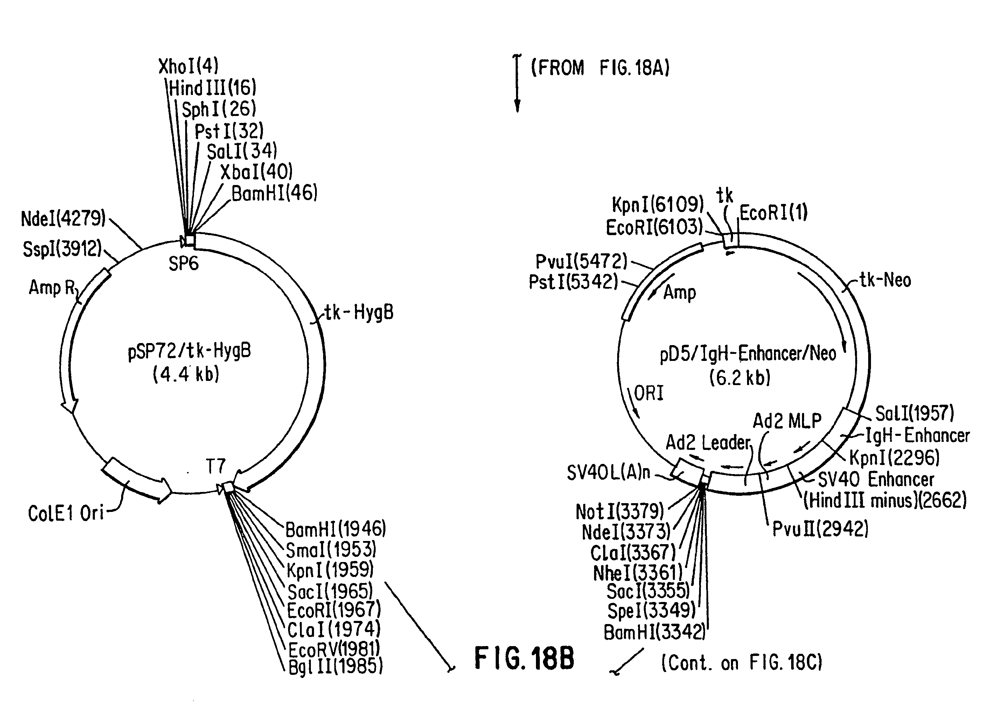

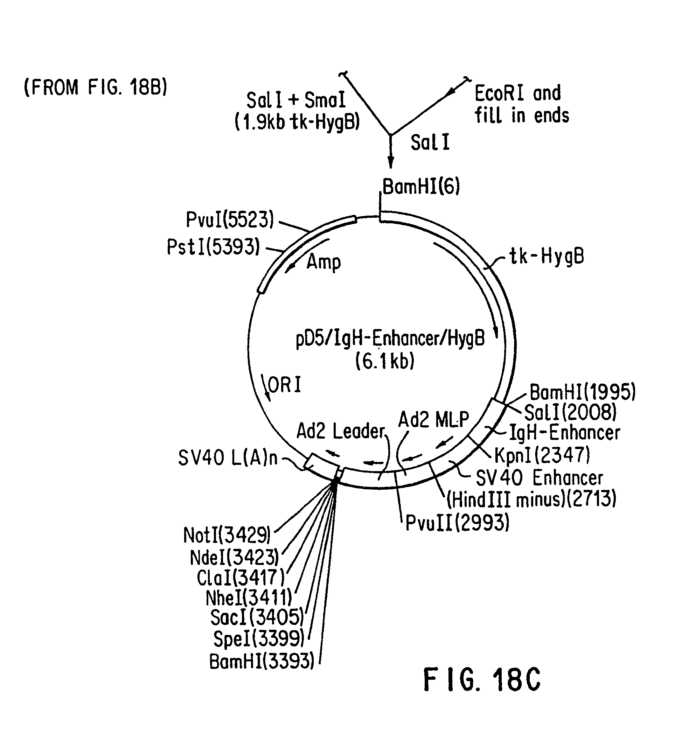

Expression of the 1B4 CDR-grafted kappa light chain was accomplished by transferring this cistron from its position within the pSP72 intermediate vector to the hygromycin B selectable eukaryotic expression vector (Figure 18). A 1.5 kb DNA fragment resulting from the endonuclease digestion of p8952 with SpeI and ClaI was purified by agarose gel electrophoresis and ligated into the expression vector (p8942) which had previously been linearized, by digestion with the same two restriction enzymes and agarose gel purified.

The heavy chain eukaryotic expression vector (p8958) was constructed in two steps (Figure 11). First, p8949 containing the modified heavy chain variable region of murine 1B4 was digested with Bgl II and Bam H1. The agarose gel purified 0.8 kb fragment was ligated into the BamH1 site of the p8941 vector and recombinants containing this fragment in the proper orientation were identified. One such plasmid was linearized by BamH1 digestion and ligated with the 1.8 Kb BamH1 fragment representing a short version of the human gamma 4 constant region derived from plasmid p8947 by PCR amplification as described above. Following the identification of clones containing these inserts in the appropriate orientation plasmid DNAs were grown (Maniatis et al., supra) and purified for transfection into recipient mammalian cells (Maniatis et al., supra; Birbion and Doly, supra.

Equal amounts (10µg) of the plasmids encoding the chimaeric IgG4 heavy chain and the 1B4 CDR-grfted kappa light chain were transfected by standard calcium phosphate precipitation procedures into human 293 cells, and the monkey cells COS-7 and CV-1P. The culture supernatant fluids were assayed by a trapping Elisa (described below) for the secretion of a human IgG4/kappa immunoglobulin.

An Elisa was developed for the quantitation of the amounts of a 1B4 recombinant antibody expressed in conditioned mammalian cell growth medium. Immulon-2 (Dynatech Labs.) 96-well plates are coated overnight with a 5 µg/ml solution of mouse anti-human kappa chain constant domain monoclonal antibody (cat. #MC009, The Binding Site, Inc., San Diego, CA) in 0.1 M NaHCO₃ buffer (pH 8.2) at 4° C, and blocked with 1% bovine serum (BSA) in 0.1 M NaHCO₃ for 1 h at 25° C. After this and all subsequent steps, washing was perfomed with phosphate buffered saline (PBS). The wells are then inoculated with conditioned medium containing recombinant anti-CD18 antibody, or with predetemnined quantities of human IgG4/kappa purified by protein A Sepharose (Pharmacia Fine Chemicals) chromatography from human IgG4 myeloma serum (cat. # BP026,The Binding Site, Inc.) All samples are diluted in PBS containing 0.05% Tween-20. 100 µl aliquots are incubated for 1 h at 37° C in triplicate, and standard calibration curves are constructed using IgG4 concentrations ranging from 10 ng/ml to 100 ng/ml. Bound and fully assembled human IgG4 (either native or recombinant 1B4-human IgG4 constructs) is detected with 100 µl aliquots of a 1:500 dilution of mouse anti-human IgG4 Fc monoclonal antibody conjugated to alkaline phosphatase (cat #05-3822, Zymed Laboratories, Inc.) in phosphate buffered saline (PBS) containing 1% BSA. After incubation for 1h at 37°C and subsequent washing, the quantities of bound conjugate are detected by incubating all samples with a 1 mg/ml solution of p-nitrophenyl phosphate in 0. 1 M 2,2′ amino-methyl-propanediol buffer, pH 10.3, for 30 min at 25°C. The adsorbance of the wells is determined with a UV Max ELISA plate reader (Molecular Devices) set at 405 nm. All supernatant fluids from the transfected cells are found to contain this immunoglobulin, though in various amounts (Figure 12). The antibody secreted by the transfected 293 cells is concentrated by protein A chromatography and the concentrations of the recombinant human anti-CD18 antibodies determined by the trapping Elisa described above, are used to compete with the binding of radiolabeled murine 1B4 to the CD18 ligand on the surface of activated human PMNs. Affinities of various r-h-anti-CD18 antibody constructs are determined using a competitive ¹²⁵I-1B4 soluble binding assay with stimulated human polymorphonuclear leukocytes (PMNs). Purified murine anti-CD18 monoclonal antibody (50 µg) is iodinated using chloramine-T (Hunter, W.M. and Greenwood, F.C., Nature 194: 495-496, 1962), and the radiolabeled antibody purified using a Bio-Sil TSK250 (Biorad, Richmond, CA) gel filtration HPLC column (which fractionates proteins in the range of 1-300 x 10³ daltons) equilibrated in 0.1 M phosphate buffer, pH 7.0. Effluent radioactivity is monitored with an in-line detector (Beckman Model 170; Beckman, Fullerton,CA) and total protein measured at OD₂₈₀ with a Kratos Spectroflow 757 detector (Kratos, Mahwah, N.J.). A single ¹²⁵I-1B4 peak composed of coincident OD₂₈₀ and radioactivity tracings characteristically elutes 6 minutes, 30 seconds following sample injection. Specific activity of the product is generally about 10 µCi/µg protein, and 97-99% of the counts are precipitable with 10% trichloroacetic acid. The binding of this radiolabeled antibody is assessed on human PMNs purified on a discontinuous Ficoll/Hypaque gradient (English and Anderson, J. Immunol. Methods 5: 249-255, 1974) and activated with 100 mg/ml phorbol myristate for 20 minutes at 37°C (Lo et al., J. Exp. Med. 169: 1779-1793, 1989). To determine the avidity of antibodies for CD18 molecules on the PMN surface, about 1 x 10⁵ activated PMNs are incubated in a buffer such as Hanks balanced salt solution containing 20 mM Hepes (pH 7.2), 0.14 units aprotinin (Sigma Chemical Co.) and 2% human serum albumin (binding buffer) containing 1.3 ng ¹²⁵I-1B4 (2.8 x 10⁻¹¹ M) in the presence of increasing concentrations of unlabeled 1B4 antibody (10⁻⁷ to 10 ⁻¹⁵ M) in a 300 µl reaction volume for 1 h at 4°C with constant agitation. Cell bound 1B4 was separated from the unbound antibody by centrifugation through a 0.5M sucrose cushion (4,800 x g, 3 minutes); the tubes are frozen on dry ice, and the tips cut off and counted with an LKB gamma counter. The 1C₅₀ of the anti-CD18 antibody for the inhibition of ¹²⁵I- 1B4 antibody binding is calculated using a four parameter fitter program (Rodbard, Munson, and DeLean, in "Radioimmunoassay and Related Procedures in Medicine", International Atomic Energy Agency, Vienna, vol I, 469 - 504, 1978). The affinity of the various r-h-anti-CD18 antibodies for the CD18 ligand is detemnined in a similar manner using murine ¹²⁵I-1B4 antibody and increasing quantities, as determined by the trapping Elisa, of unlabeled r-h-antiCD 18. The results of the binding assays are shown in Figure 13 and indicate that the avidity of the chimeric heavy chain/grafted light chain recombinant 1B4 antibody (circles) is approximately that of the murine 1B4 monoclonal antibody (diamonds).

### EXAMPLE 2

### Preparation of Fully Grafted Recombinant Human IgG4 Antibodies

This example shows the production of recombinant human IgG4 antibodies, whose variable domains contain the CDR residues of the murine monoclonal antibody 1B4. The construction of the CDR-grafted light chain framework derived from the human sequence of REI fused with a human kappa light chain constant region was described in the preceding example (Example 1).

The 1B4-specific heavy chain component of the recombinant antibody was constructed from the IgG4 heavy chain constant region, described in Example 1, fused to a pre-selected human heavy chain variable region framework sequence into which the 1B4 CDR residues were transplanted. The murine 1B4 MAb m1B4 heavy chain was first analyzed to determine the precise position of the CDR sequences. These were determined by visual comparisons with the data sets found in Kabat, Wu, Reid-Miller, Perry, and Gottesman, *Sequences of proteins of immunological interest.* (US Dept Health and Human Services, Bethesda, MD, 1987). Once the boundaries of the CDRs were determined these sequences were removed to leave the murine FRs alone. This sequence was then used to query the human immunoglobin database which was mainly derived from release 22 of the PIR database ( George et al., Nucl. Acids Res. 14: 11-16 (1986)). The sequence search was performed using the Profile search system of the GCG sequence analysis package (Devereux et al., Nuc. Acids Res. 12: 387-395[1984]). The matrix used for similarity comparisons was the Dayhoff evolutionary distance matrix (R. M. Schwartz, M. O. Dayhoff, in *Atlas of Protein sequence and* *structure* M. O. Dayhoff, Eds. (National Biomedical Research Foundation, Washington, DC, 1979)). Additionally, the Risler structural distance matrix (Risler et al., J. Mol. Biol. 204: 1019-1029[1988]) was used to generate the murine sequence profile, and the results of searches with this query were considered with those generated using the Dayhoff matrix. Use of the profile searching system also allowed the weighting of specific residues within the murine FR that were deemed important based on various criteria. The sequences that repeatedly showed the highest levels of sequence similarity in the database queries were then analyzed using a pairwise comparison to the FRs of murine 1B4. The program Gap of the GCG package was used for this analysis, because it produces an exact measure of both the sequence similarity and identity shared between two sequences. This method was used to select the human sequences Gal and Jon, which shared a similarity of 85% and 88% and identities of 79% and 75% with murine 1B4, respectively (Figure 14). To prepare a recombinant DNA representing the 1B4 heavy chain CDRs within each of these frameworks the following procedures were performed.

Two sets of four long oligonucleotides were synthesized. When each set was combined, they encoded that portion of heavy chain corresponding to the murine 1B4 variable region present in the chimaeric heavy chain expressed in Example I. The four oligonucleotides of each set (Figure 15, Figure 16), 1 pmole of each, were combined in a standard PCR reaction with 2.5 units of Taq polymerase and 50 pmoles of each terminal amplifying oligodesoxynucleotide (Figure 15, Figure 16). By virtue of the complementary ends of the single-stranded oligonucleotides, the polymerization-denaturation-polymerization cycles of the polymerase chain reaction result in the formation, and subsequent amplification, of the combined sequences. Following 25 cycles of amplification the combined 0.4 Kb fragment was electrophoretically purified and extracted from an agarose gel. In parallel, two DNA fragments representing amino terminal sequences encoding the signal peptide and carboxy terminal sequences encoding framework 4, splice donor, and intronic sequences were amplified using oligodeoxynucleotide primer pairs (Figure 15) and the M13VHPCR1 plasmid DNA template described in example I. These two DNA fragments were purified by agarose gel electrophoresis, as above, and 10 ng of each was combined with 10 ng of the amplified variable region fragment, 2.5 units of Taq polymerase, 50 pmoles of terminal primers (Figure 15) and the mixture was amplified by 25 cycles of PCR. The resultant 0.8 Kb fragment was digested with restriction enzymes Spe I and BamH1 (Gal) and Hind III and Bam H1 (Jon). Following agarose gel electrophoresis, the purified DNA fragment was ligated into the heavy chain expression vector, p8958, in place of the chimaeric variable region (Figure 11). In this way, two unique heavy chain frameworks containing grafted 1B4 CDRs (1B4/Jon and 1B4/Gal) were constructed. Each fully grafted heavy chain expression vector plasmid was co-transfected with the fully grafted 1B4/REI light chain expression vector (Example 1) plasmid into 293 cells and the antibody present in conditioned medium was isolated by protein A chromatography. The recombinant humanized 1B4 (h1B4) avidity of these two antibodies for the CD18 ligand displayed on the surface of activated human PMNs was compared with that of the chimaeric/grafted antibody described in Example I. Figure 20 shows that although each hetero-dimeric antibody contains the same set of six CDRs, they do not exhibit identical avidity for the ligand. Thus, the biological properties of an antibody molecule (ie., its avidity) rely significantly on the variable region framework structure which support the CDR loops.

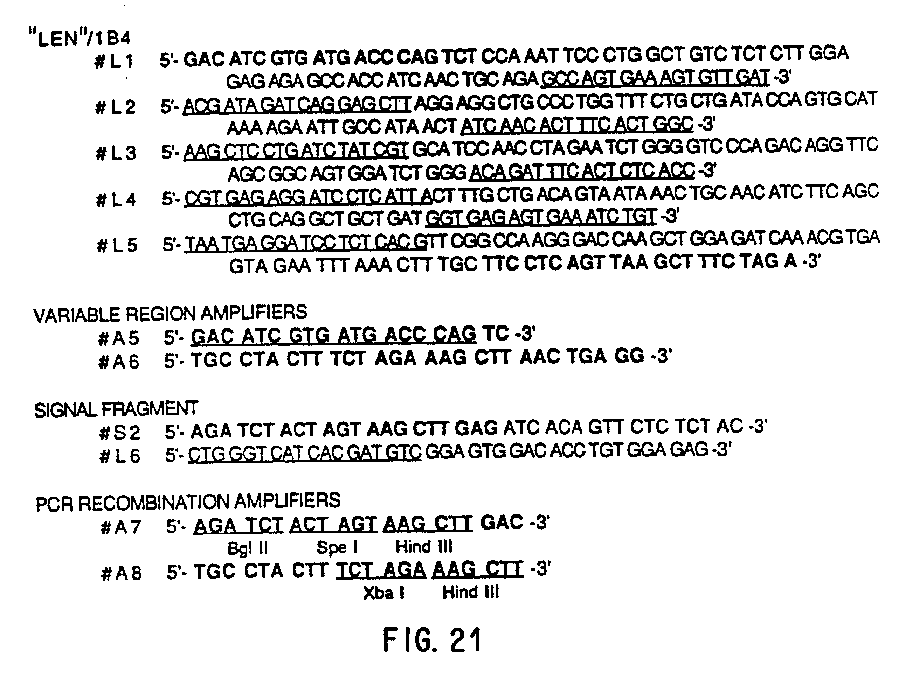

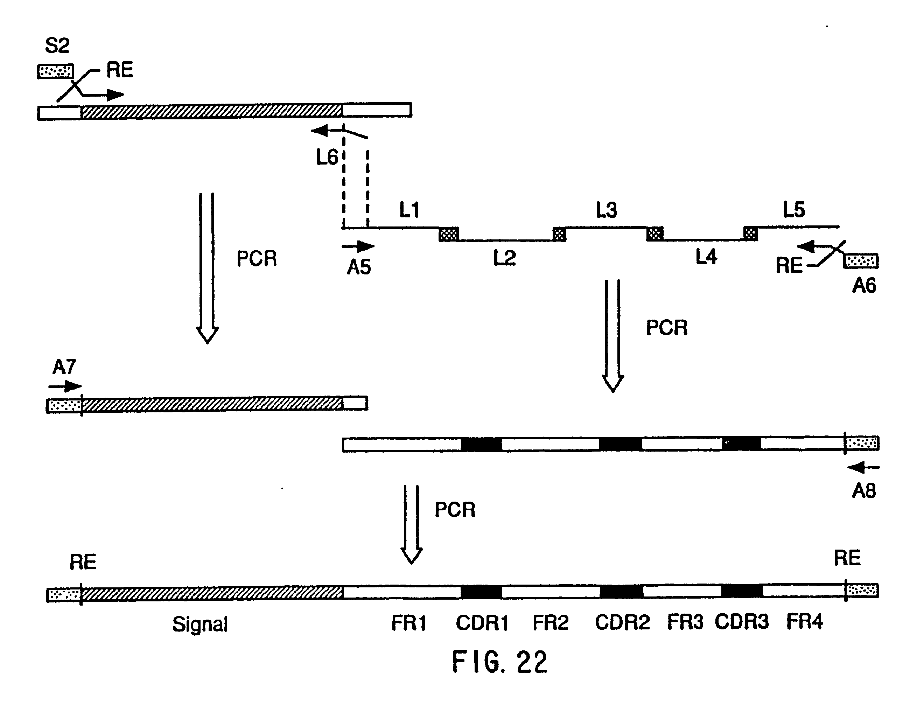

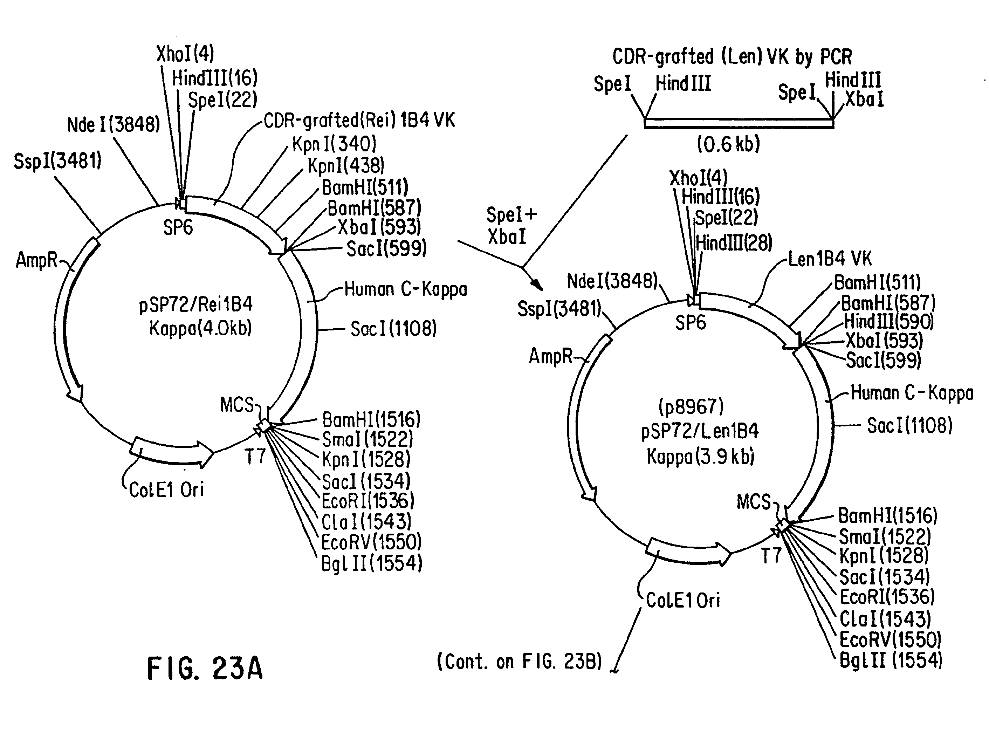

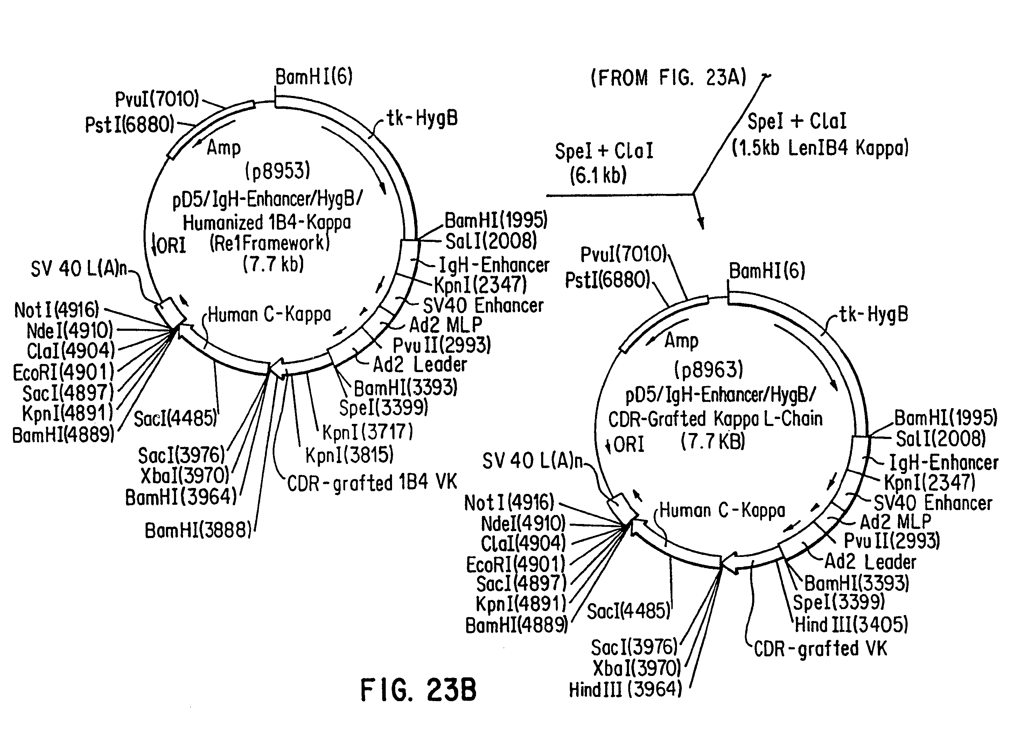

To determine the relative contribution of the light chain variable region to the enhanced avidity of the Gal/REI grafted hetero-dimer a second light chain framework was constructed containing the 1B4 CDR sequences. The light chain framework Len was identified as a donor framework sequence based upon its selection from the database. Len was identified by using the murine 1B4 light chain framework sequence, with CDRs removed based upon visual identification of the CDRs when compared to Kabat (supra), to query the human immunoglobin database. The methodology of the query was similar to that described for the heavy chain FRs. Len was shown, by Gap analysis, to be 90% similar and 81% identical to the murine 1B4 light chain FR. Len was thought to be a better choice for grafting of the light chain CDRs than REI, based on its higher levels of both similarity and identity to 1B4 as compared to REI (82% similarity and 65% identity) (see Figure 14). A set of five long oligodeoxynucleotides (Figure 21) representing the Len light chain framework with 1B4 specific CDR sequences and intronic sequences were synthesized using 2.5 units of Taq polymerase and 50 p moles of each terminal amplifying oligodeoxynucleotide primer and combined by PCR, as described above for the Jon and Gal frameworks (Figure 22). Following 25 cycles of amplification the combined 0.6 kb DNA fragment was purified by agarose gel electrophoresis. In parallel, a DNA fragment representing the amino-terminal signal peptide was amplified using a oligodeoxynucleotide primer pair (Figure 21) and the M13VHPCR1 plasmid DNA template, as described in Example 1. This fragment was also purified by agarose gel electrophoresis. These two DNA fragments are placed together, 10 ng of each, with 2.5 units of Taq polymerase, 50 p moles of terminal oligodeoxynucleotide primers (Figure 21) and the entire mixture is subjected to 25 cycles of PCR amplification. The resultant 0.8 kb DNA fragment is digested with restriction enzymes Spe I and Xba I, purified following agarose gel electrophoresis, and ligated into the pSP72/REI 1B4 intermediate vector which is digested with the same two restriction enzymes and electrophoretically purified from its liberated REI/1B4 variable region containing DNA fragment (see Figure 23). The combined light chain variable region and kappa constant region within a sequence verified clone (p8967) is excised by digestion with restriction enzymes Spe I and Cla I and this 1.5 kb agarose gel electrophoretically purified DNA fragment is cloned into the light chain expression vector p8953, after this latter plasmid is electrophoretically purified from its REI/1B4/kappa light chain insert following digestion with both Spe I and Cla I restriction enzymes. The fully CDR-grafted Gal/1B4 heavy chain expression vector and the fully CDR-grafted Len/1B4 or REI/1B4 light chain expression vector DNAs (10 ug each) are co-transfected into 293 cells and the antibody present in conditioned medium 48 hours later is isolated by protein A Sepharose chromatography. The avidity of these two recombinant antibodies for the CD18 ligand present on the surface of activated human PMNs is determined and compared to that of the murine 1B4 MAb (Figure 20). The differences between the two humanized 1B4 recombinant antibodies for the ligand, as measured by their IC₅₀s, revealed that a compairson of p values between Gal/Rei and Gal/Len are statistically significant by the students umpaired t-test bus the standard deviations of both Mabs overlap (see Figure 20). Thus, although the Len light chain variable region framework sequences, relative to the REI light chain frameworks, show more identical residues and more similar residues when aligned to the murine 1B4 frameworks, this has little, if any, impact on the antibody/antigen interactions measured by avidity. Comparison of the presumed three dimensional structure of these two light chain variable regions (REI and Len) indicates that the alpha carbon trace of the 1B4 CDRs residing within these frameworks are superimposable, again suggesting that the both frameworks identically support the CDRs in space. Does the 1B4 heavy chain variable region play a greater role in avidity of the antibody for its ligand? To address this question, and also to investigate the role of a small number of heavy chain variable region framework sequences, modifications of the Gal/1B4 fully grafted molecule are performed.

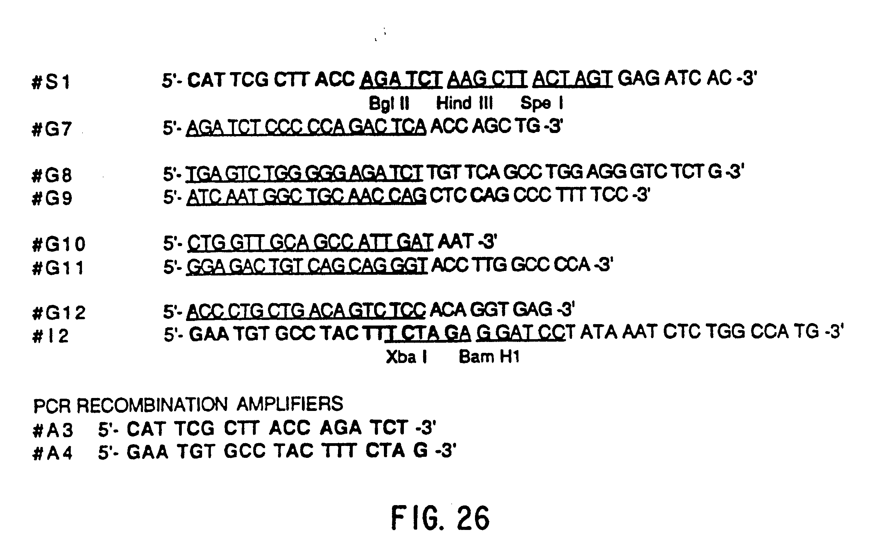

Three residues within the heavy chain variable region of Gal/1B4 are chosen to mutate such that they become identical to their counterparts in the murine 1B4 framework (see Figure 14). To accomplish the mutation of three well separated residues simultaneously the following procedures are performed. Four oligodeoxynucleotide primer pairs (Figure 26) are synthesized which incorporate the deoxynucleotide alterations necessary to mutate the amino acid residues located in FR1, FR2, and FR4 of the Gal/1B4 DNA template. In this instance, the polymerase chain reactions needed to produce four overlapping DNA framents were amplified in such a way as to generate primarily single-stranded DNAs representing the outside two DNA framents, while the inside two DNA fragments are amplified so as to produce double-stranded DNAs. This approach of combining four amplified DNAs is facilitated by the above modification and, when combined with the use of terminal amplifying oligodeoxynucleotide primers which are unique to residues found only in the outside amplified DNA fragments, remove the need to purify the PCR products between the first and second round of amplification. Thus, asymmetric PCR is used to amplify the two terminal DNA fragments. Combined into the standard PCR amplification reactions are 50 p moles of primer #S1 and 0.5 p moles of primer #G2 (Figure 26) or 50 p moles of primer #I2 and 0.5 p moles of primer #G2 (Figure 26) and the Gal/1B4 containing plasmid DNA template (10 ng/reaction), 2.5 units of Taq polymerase, and the remaining standard reaction components. The two internal DNA fragments are amplified using the standard procedures which include the presence of 50 p moles of each of the oligodeoxynucleotide primers, 2.5 units of Taq polymerase, and the same template DNA and reaction components described above. Following 25 cycles of amplification (as described previously) the reactions are made to contain 1 ml of H₂O, and each is placed in a Centricon 100 cartridge (Amicon, Danvers, MA), centrifuged for 30 minutes at 3500 x g, at 4° C, and the retentate is resuspended in another 1 ml of H₂O and the centrifugation is repeated. The final retentate is resuspended in 100 µl of H₂O. Each of the four reaction products is combined (1 µl of each of the retained DNA solutions), the standard components are added, 2.5 units of Taq polymerase, and 50 p moles of the PCR recombination amplifying primers (Figure 26), and the reaction is cycled 25 times. The resultant 0.8 kb DNA fragment is phenol extracted, concentrated by ethanol precipitation, and digested with Spe I and Bam H1 restriction enzymes. Following purification of this 0.8 kb DNA fragment by agarose gel electrophoresis it is cloned into the heavy chain expression vector p8958, after this latter plasmid is electrophoretically purified from its Gal/1B4 heavy chain variable region insert liberated by digestion with both Spe I and Bam H1 restriction enzymes. The fully CDR-grafted Gal-m1/1B4 heavy chain expression plasmid DNA is co-transfected (10 ug of each DNA) with the fully CDR-grafted REI/1B4 light chain expression plasmid DNA or the fully CDR-grafted Len/1B4 light chain expression plasmid DNA into 293 cells. The resultant antibodies present in the conditioned medium 48 hours later are isolated by protein A sepharose chromatography and subjected to avidity measurements. Independent of the origin of the light chain variable region framework, the measured avidity for CD18 on the surface of activatived human PMNs of the two antibodies is nearly identical. Again the role of the light chain variable region frameworks seems to be minimal. The avidity of the mutated Gal framework (mutated Gal/Rei, Figure 20) is significantly improved relative to the non-mutated Gal heavy chain framework (Gal/Rei in Figure 20) and its avidity is nearly equivalent to that of native m1B4 (Figure 20). It is concluded that one or more of the three residues mutated contributes to the display of the CDRs (antigen binding sites), thus proper framework choice is critical for optimal humanization of recombinant antibodies. Indeed, it appears that the framework closest to the CDRs dictates the final structural arrangement of the CDRs and thus the ability to bind antigen. Additional comparisons of the heavy chain frameworks reveal major differences between those of New and Jon or Gal when the packing residues are examined (Figure 14). Packing residues as used herein is defined as internal or non-surface exposed residues of the structure that may be involved in intrastrand or interstrand forces. These packing residues are associated with the framework regions adjacent to the CDRs and are involved in the proper orientation of the CDRs for interaction with the substance that induced the antibody formation. Only 27 of41 internal residues of New match the corresponding residues in the murine 1B4 framework. This is contrasted to the match of 38 of 41 residues by the human Gal framework. The localization of the region of greatest variation to those residues ending framework 2 may explain the differences between the Gal and Jon supported antibodies. This region of framework 2 is where these two differ and where Gal-M1 differs from Gal.

### EXAMPLE 3

### Enhanced Expression Systems

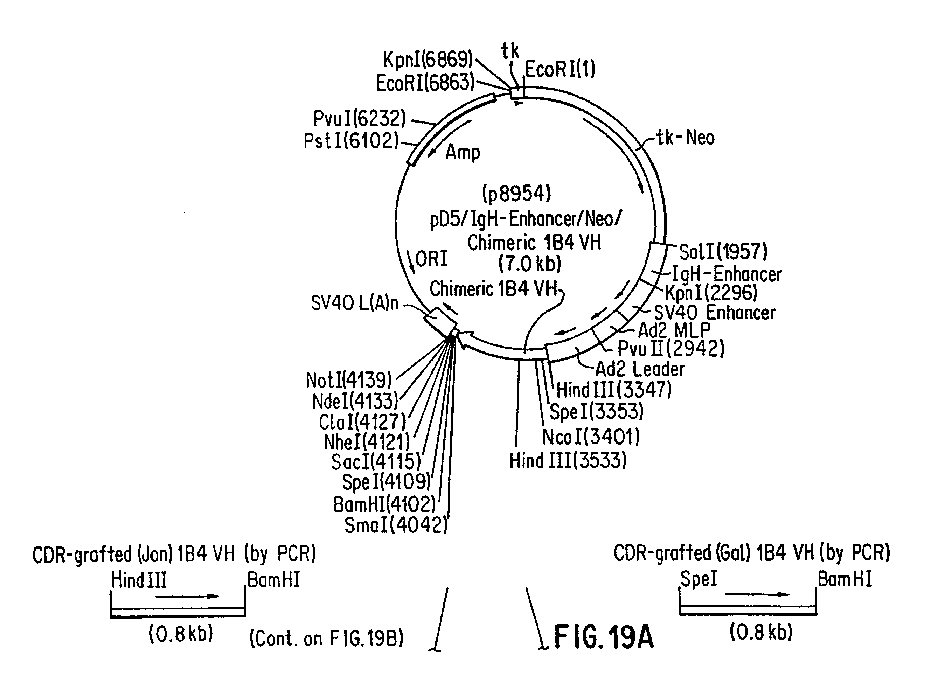

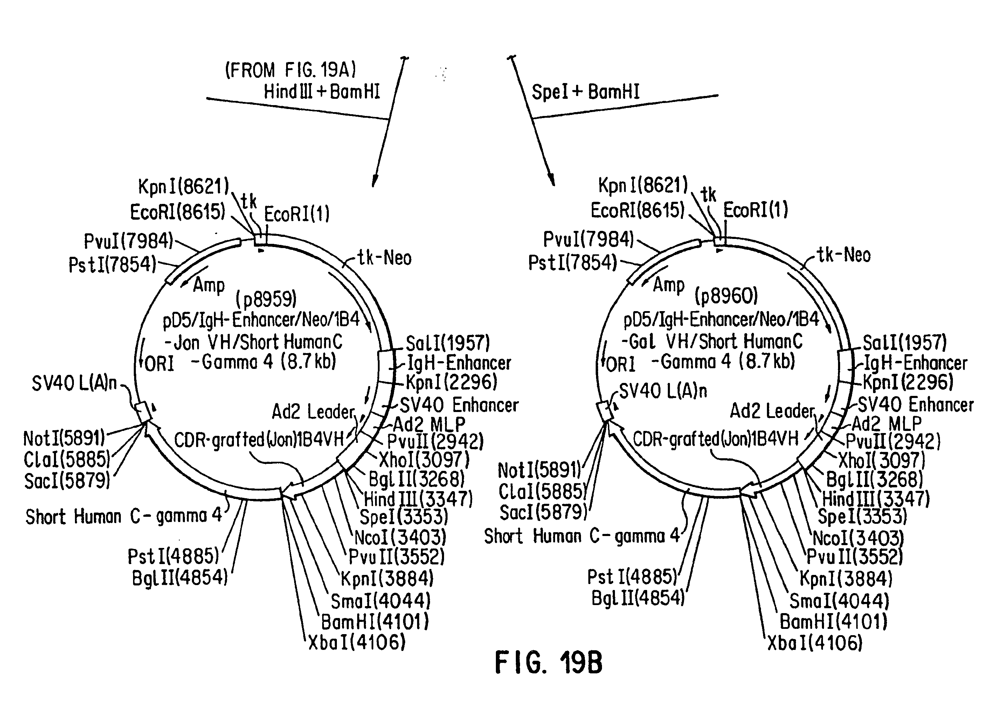

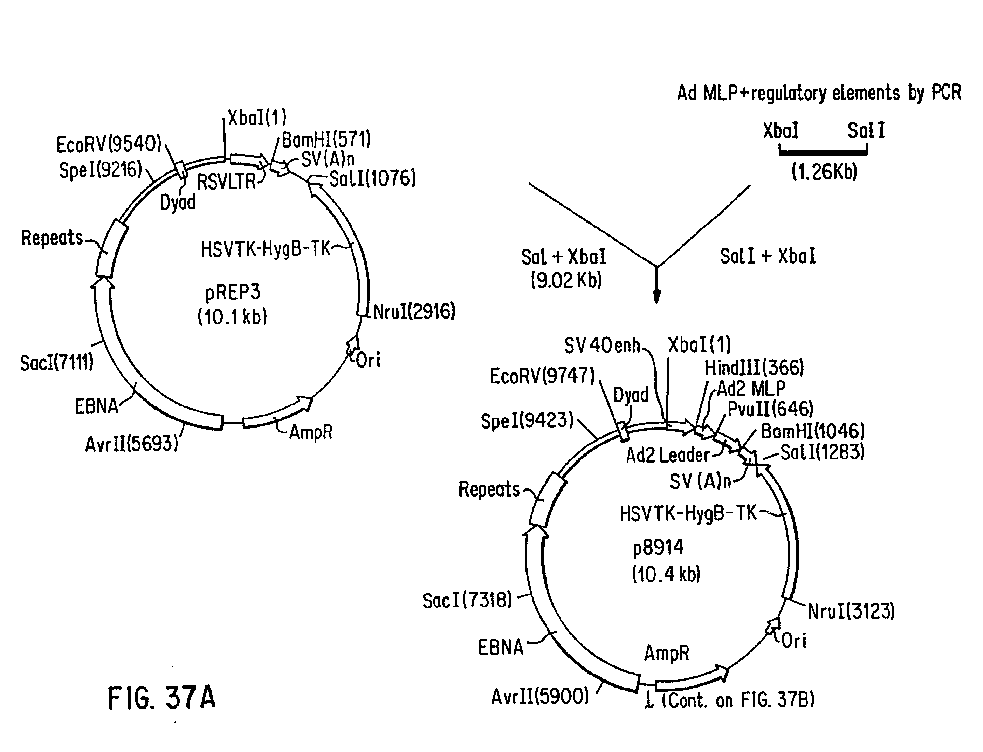

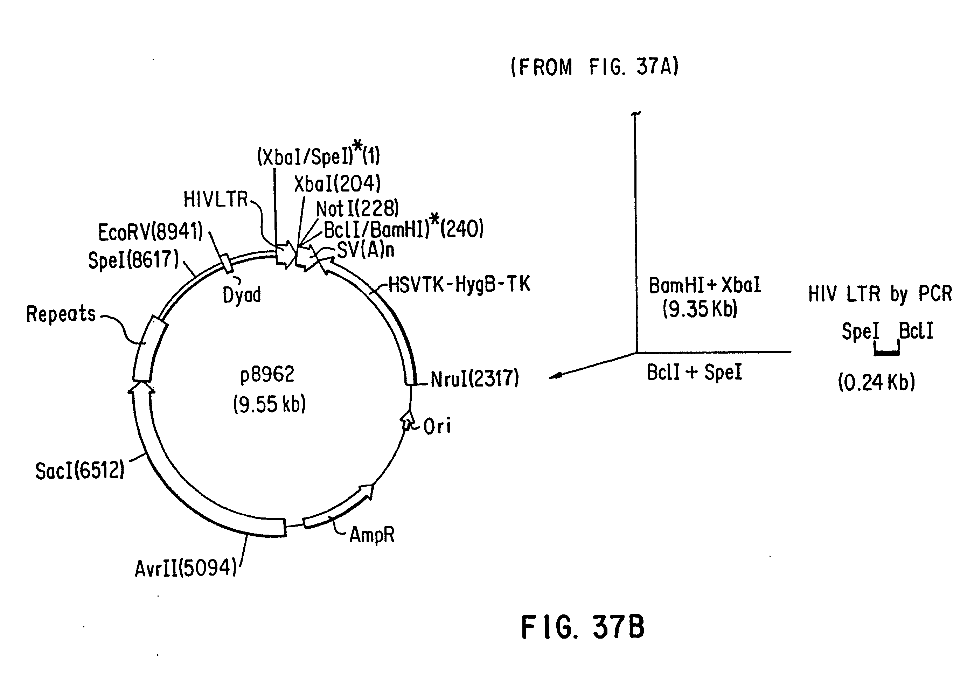

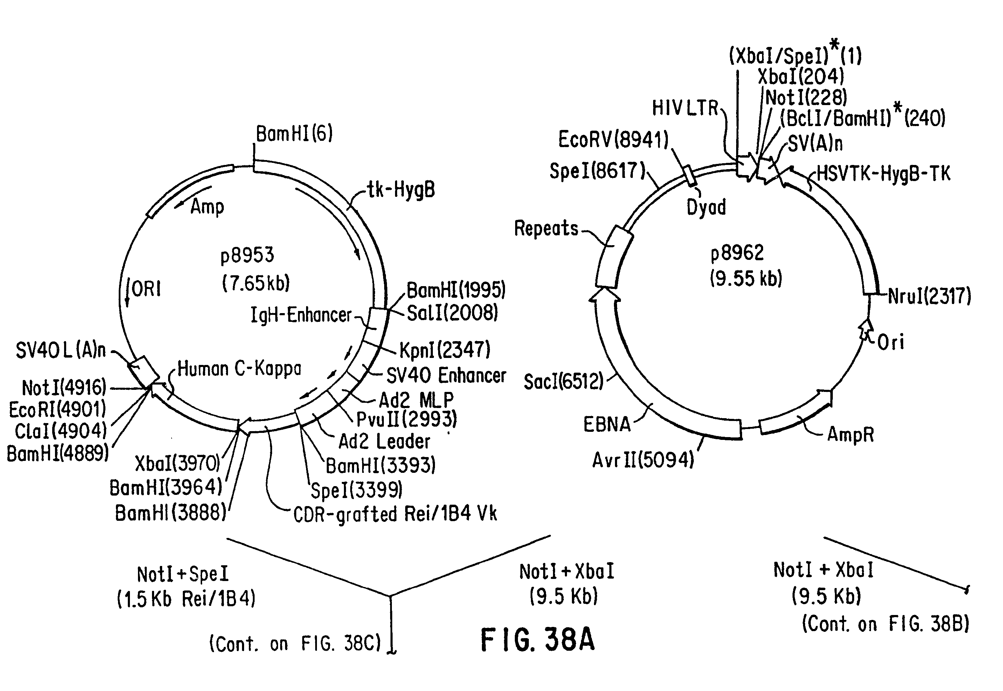

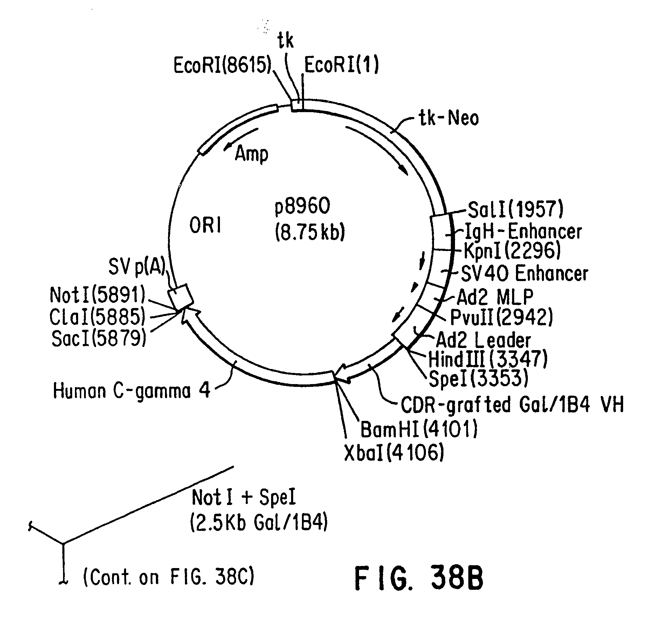

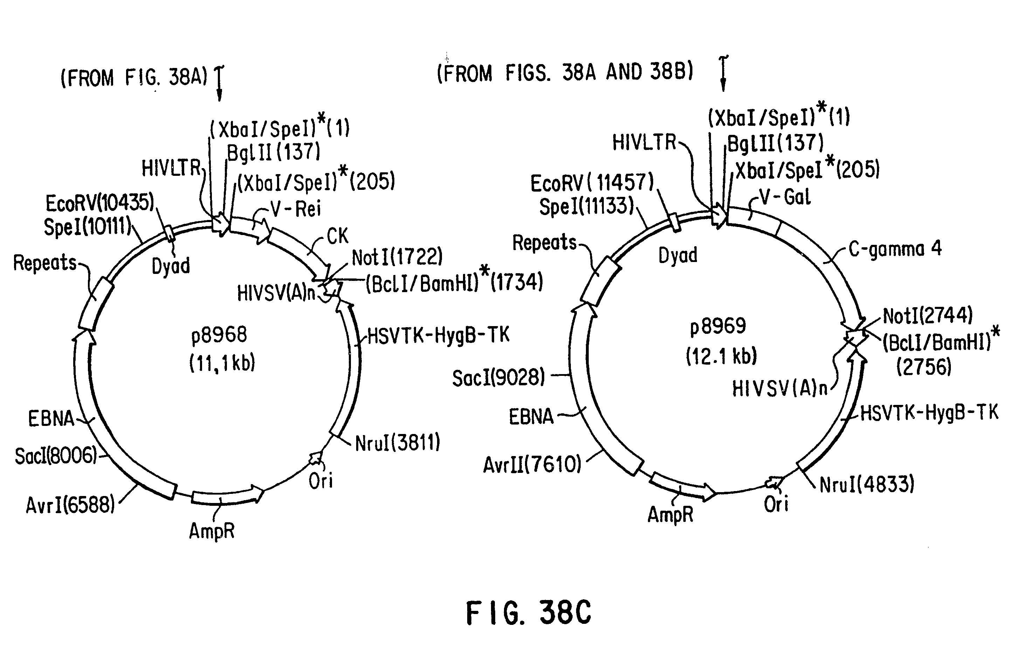

This example shows expression systems employed to produce large quantities of recombinant CDR-grafted IB4 antibodies as discussed in Example 2. The first expression system applicable to many mammalian cells utilizes the extrachromosomal characteristics of EBNA- 1 /oriP based DNA plasmids (Yates et al., Nature: 313: 812, 1985). Such a vector, pREP3 described by Hambor et al. (Proc. Natl. Acad. Sci. USA 85: 4010, 1988), containing the hygromycin B selection cassette and the Rous Sarcoma Virus (RSV) LTR for transcription of the gene of interest was modified as disclosed. The RSV LTR, as well as the poly A addition signal, was removed by digestion of the pREP3 plasmid DNA with Sal I and Xba I followed by agarose gel purification of the 9.02 Kb promoterless fragment. DNA from plasmid pD5mcs (see Figure 10), containing the adenovirus major late promoter, a multi-cloning site, and SV40 poly A addition signal was used as the template for the PCR amplification of those sequences beginning with the SV40 enhancer and ending with the SV40 poly A addition signal. In the process of amplification Xba I and Sal I restriction enzyme sites were appended to the product ends by their incorporation into the synthetic PCR oligodeoxynucleotide primers. The expected 1.26 Kb PCR amplified product was agarose gel purified following its digestion with Xba I and Sal I restriction enzymes and ligated into the 9.02 Kb EBNA/oriP backbone vector. The resultant plasmid (p8914) constitutes a versatile mammalian expression vector into which can be ligated either the heavy chain or light chain expression cassette contained within plasmid p8958 (see Figure 19) or p8953 (see Figure 6), respectively. The p8914 plasmid was also the template for the HIVLTR promoter version of the EBNA/oriP backbone vector. In order to switch to the HIVLTR promoter the p8914 plasmid DNA was digested with Bam H1 and Xba 1. The 9.35 Kb promoterless backbone was purified by agarose gel electrophoresis. The HIVLTR promoter, from residue -117 to +80 (as found in the vector pCD23 containing this portion of the HIV-1 LTR; (Cullen, Cell 46: 973[1986]) was PCR amplified from the plasmid pCD23 using oligodeoxynucleotide primers which appended to the ends of the product the Spe I and Bcl I restriction sites. Following the digestion of the resulting 0.24 Kb PCR product with these latter enzymes the fragment was agarose gel purified and ligated into the 9.35 Kb DNA promoterless DNA fragment described above. The p8962 plasmid so constructed was also the recipient of the heavy and light chain cassette (Figure 37). To accomplish this the p8962 plasmid DNA was digested within its multicloning site with Not I and Xba I so as to linearize the DNA. The 9.5 Kb linearized expression vector DNA was ligated to either the 2.5 Kb heavy chain cassette obtained by agarose gel purification of Not I and Spe I digested p8960 DNA or the 1.5 Kb light chain cassette obtained similarly following digestion of p8953 DNA with Not I and Spe I. These constructed EBNA/oriP based expression vectors,p8969 and p8968, (Figure 38) were co-transfected into CV1P cells (monkey kidney cells; Figge et al., Cell 52: 713[1988]) which constitutively express the HIV-1 TAT protein by virtue of having previously been transfected with the plasmid pMLTAT (Siekevitz et al., Science 238: 1575[1987]). The cell clones which arose in DMEM medium containing 10% heat inactivated newf born calf serum, 200 µg/mL of G418, and100 µg/ml of hygromycin B were picked using cloning cylinders (Fishney, In, Culture of Animal Cells, Alan R. Liss, Inc. New York, 1983) and expanded individually. Clones were screened for the secretion of recombinant antibody using the ELISA assay previously described. Multiple cell clones were expanded and their antibody secretion levels were determined to be in ther range of 75 ng - 2 µg of antibody per 96 hours of medium conditioning of 6 well plate cultures. The most productive of these clones was eventually adapted to growth on microcarriers (cyledex 3 and cultisphere GL) and produced approximately 100 mg/L of recombinant antibody each 3 day harvest in serum-free medium at a cell density of 1-2 x 10⁶ cells per ml.

### EXAMPLE 4

### In Vitro Activity Of Recombinant Human Anti-CD18 Antibodies

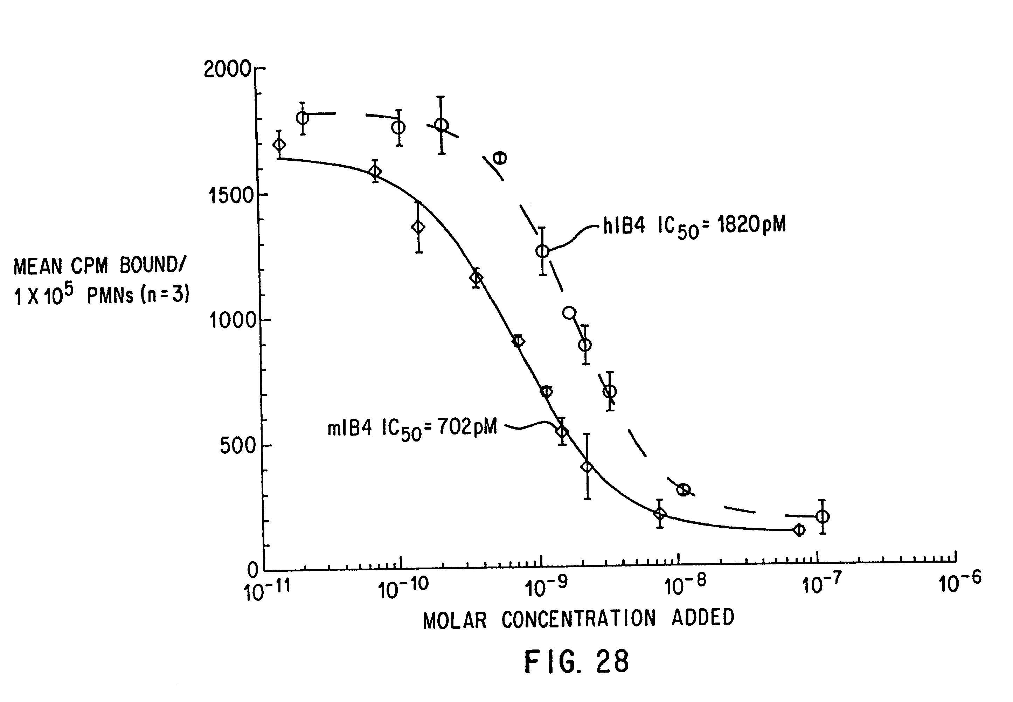

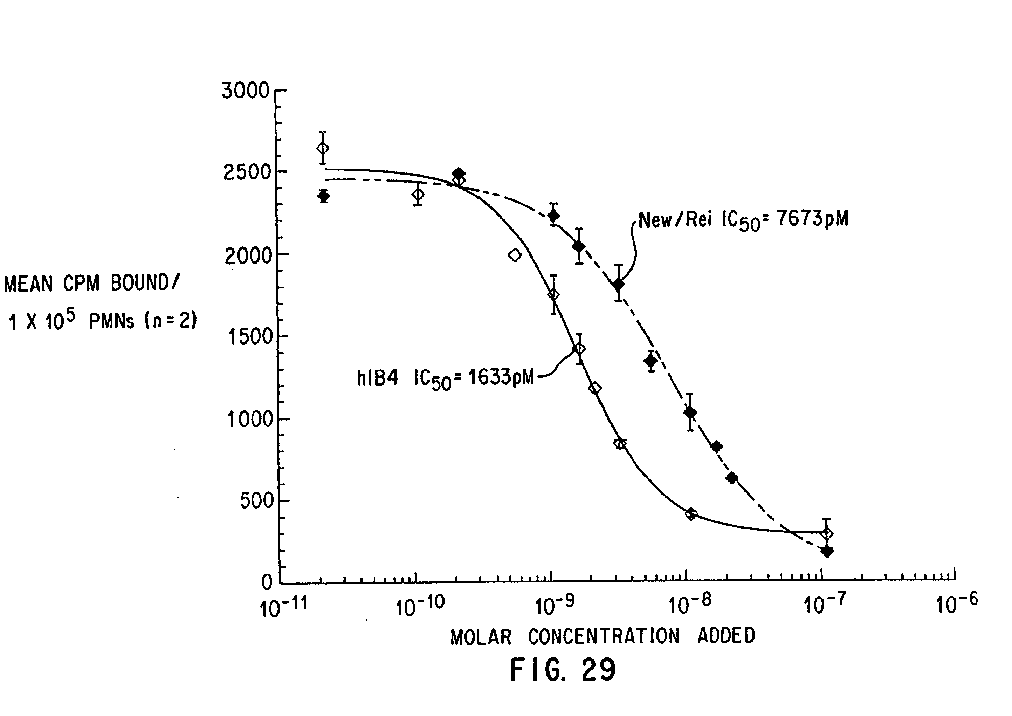

To increase the precision of avidity determinations, the IB4 competitive binding assay of Example 2 was modified as follows. Both mIB4 (50 µg) or hIB4 (from Example 3) were iodinated using chloramine-T, and the radiolabeled IgG purified over a Bio-Sil TSK250 (Biorad) gel filtration HPLC column that fractionates proteins in the range of 5-300 x 10³ daltons. Effluent radioactivity was monitored with a Beckman #170 in-line gamma counter (Beckman, Fullerton, CA), the total protein was detected by absorbance at 280 nm with a Kratos Spectroflow 757 detector (Kratos, Mahwah, NJ), and the column was equilibrated with 0.1 M phosphate buffer (pH 7.0). A single symmetrical peak of coincident absorbance and radioactivity tracings was routinely observed at 6 min. 30 sec. following sample injection (the retention time characteristic of IgG in this system). Specific activity of the product was usually 10 mCi/mg for m1B4 or 70mCi/mg for h1B4; 96-98% of the counts were trichloroacetic acid-precipitable in either case. SDS-PAGE and autoradiography of ¹²⁵I labeled antibody showed that 1B4 remained intact following radiolabeling. Using these radio-labeled probes, a competitive ¹²⁵I-1B4 suspension binding assay was established to determine the avidity of m1B4 or r-h-anti-CD18 (h1B4) for CD18 expressed at the leukocyte surface. Human venous blood was collected freshly into heparin (1.0 unit/ml). PMNs were purified on a Ficoll/Hypaque gradient and activated with 100 ng/ml phorbol myristate acetate in Hanks balanced salt solution containing 20 mM Hepes (pH 7.2), 0.14 units Aprotinin and 2% human serum albumin (binding buffer) for 20 min at 37°C; viability was always >95% by tryan blue exclusion following PMA activation. After washing with binding buffer, aliquots of 1 x 10⁵ stimulated PMNs were incubated in about 2-4 x 10⁻¹¹ M ¹²⁵I-1B4 in the presence of increasing concentrations of unlabeled murine or humanized 1B4 (about 10⁻¹⁵ to 10⁻⁷M) in duplicate or triplicate 300 ml volumes for 1 h at 4°C with constant agitation. The concentrations of purified radio-iodinated 1B4 or unlabeled antibody added as a competitor were determined by U.V. absorption using an E₂₈₀ of 1.35 for mIB4, 1.25 for mutant Gal/REi h1B4, and 1.30 for all other h1B4 constructs [determined by the formula E = A(Ecys) + A (Etryp) + A (Etyr) where A = the number of residues of each amino acid; Gill and von Hippel, Anal. Biochem., 182: 319-328, 1989; the E₂₈₀ of mIB4 and Gal/Rei H1B4 were also verified by quantitative amino acid analysis and differential UV spectroscopy]. After labeling, the ¹²⁵I-1B4 bound to the cells was separated from unbound antibody by underlaying each aliquot of PMNs with 250 ul 0.5 M sucrose and centrifugation (4,800 x g, 3 min.); the tubes were frozen on dry ice, and the tips cut off and counted with an LKB gamma counter. The quantity of PMN-bound ¹²⁵I-1B4 for each concentration of purified unlabeled competitor IgG was expressed as the mean CPM per 1 x 10⁵ PMNs (± SEM). 1C₅₀s for inhibition of ¹²⁵I-1B4 binding were calculated using a four parameter program ("Fitter"; Rodbard, Munson, and Delean in "Radioimmunoassay and Related Procedures in Medicine", International Atomic Energy Agency, Vienna, vol I, 469-504, 1978). The results of the binding assays are illustrated in Figures 13, 20, 28, and 29 (p values are from Student's unpaired t-test). These data indicate that: 1) the avidity of Gal/Rei h1B4 for PMN CD18 is nearly comparable to that of mIB4 (about 2-3 fold weaker); 2) the avidities of Jon/Rei and New/Rei are still weaker than that of Gal/Rei in a rank order that correlates inversely with their degree of homology relative to m1B4 frameworks; 3) the avidity of Gal/Len is nearly equivalent to the avidity of Gal/Rei; and 4) that mutant Gal/Rei and the demi-chimeric construct possess affinities apparently comparable to that of native IB4.

Inhibition of PMN attachment to human umbilical vein endothelial cell (HUVEC) monolayers.

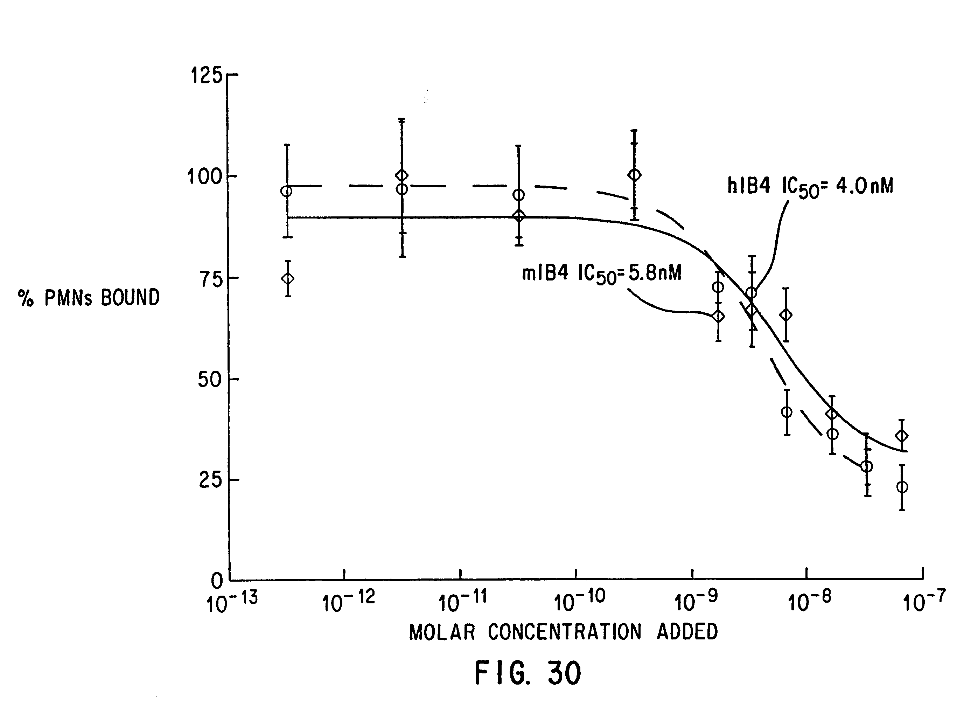

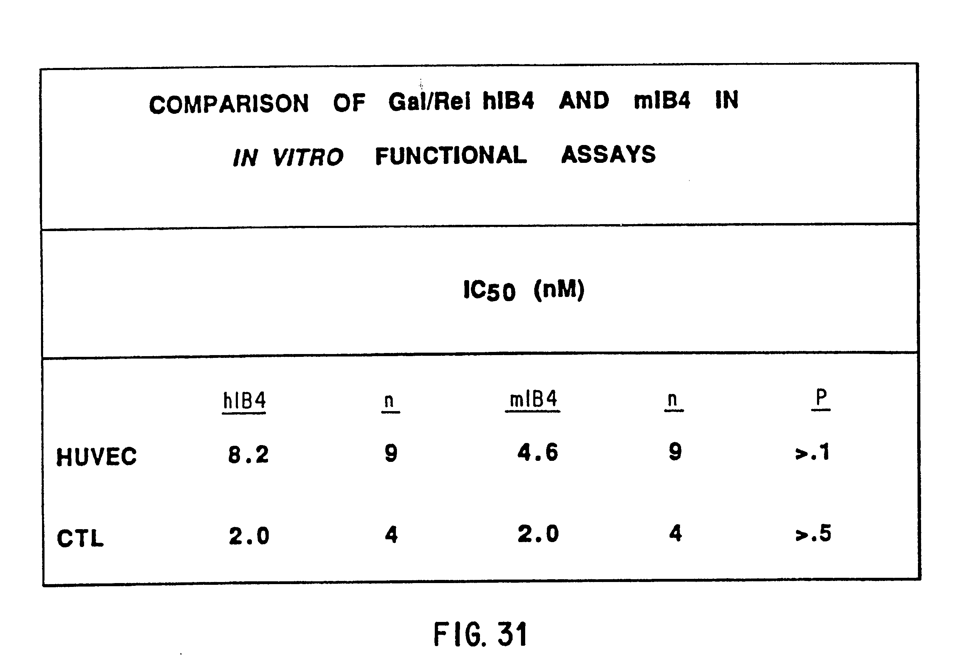

To reach tissue sites and cause inflammatory damage, PMNs must pass out of the bloodstream. This transendothelial migration depends on interaction of PMN CD18-containing receptors with ligands on and within the human endothelium. A direct expression of this process is reflected by attachment of agonist-treated PMNs to the vascular surface. To demonstrate that Gal/Rei h1B4 is a prospective anti-inflammatory agent for use in human disease, we determined whether this construct inhibits adhesion of PMA-stimulated hPMNs to quiescent human endothelial cell monolayers. Human umbilical vein endothelial cells (HUVECs) were grown in T-75 flasks coated with Vitrogen 100 (Collagen Corp., Palo Alto, CA) diluted 1:10 with PBS and dried onto the substrate. The culture medium was MCDB 107 supplemented with 15% FCS, 90 mg/ml heparin (GIBCO), and 150 mg/ml endothelial mitogen (Biomedical Technologies, Inc.); the cells were incubated in 2.5% CO₂ and 97.5% air. Cultures (passages 4-8) were dissociated with trypsin/EDTA, and the HUVECs seeded into 96-well microtiter plates (Costar) precoated with a 5 µg/ml solution of purified human plasma fibronectin in 0.1M bicarbonate (pH 8.3); these microcultures were used for the attachment assay upon reaching confluence. Human PMNs were purified from peripheral blood as described above. To measure their attachment to the HUVEC monolayers by fluorescence microscopy, PMNs were labeled with the vital fluorescent dye 1′,1′-dioctadecyl-3,3,3′,3′-tetramethylindocarbo-cyanine (DiI) (Molecular Probes, Inc.). PMNs were incubated in a 25 mg/ml sonicated solution of DiI in binding buffer for 10 min. at 37 °C, washed, and then activated with 50-100 ng/ml PMA or PDB for 10 min. at 37 °C. (These diI-labeled PMNs were tested in the competitive 1B4 binding assay to verify that their CD18 receptors were recognized by hIB4; the IC₅₀s were within the range expected for unlabeled PMNs). PMN aliquots (in quadruplicate) were pretreated with increasing concentrations of either Gal/Rei h1B4, m1B4, or the control Mab OKM-1 (associates with the CD11b component of the CR3 receptor but does not inhibit ligand binding). Incubation was performed for 15 min. at 4 °C with constant agitation, and the cells placed into the microwells containing the HUVEC monolayers (50,000- 100,000 PMNs/well). The PMNs were permitted to settle for 5 min. at 4 °C, and then incubated for 15 min at 37 °C to allow firm adhesion to occur. Unattached PMNs were removed and the cultures fixed by gentle washing with 1% formaldehyde in PBS (4 washes with an Eppendorf Plus 8 multitip pipette). The wells were filled with a solution of 5% n-propyl gallate in glycerol, and the attached PMNs counted at 195 x under rhodamine illumination with an automated Nikon Diaphot inverted fluorescent microscope fitted with an autofocus device, a customized motorized stage, and a video camera (Vidicon #8451) connected to a Model 3000 image analyzer (Image Technology Corp., Deer Park, NY) and an IBM PCXT computer. The mean number of adherent PMNs was determined for each concentration of Mab tested (± SEM), and an inhibition curve plus IC₅₀ generated with the "Fitter" program (Rodbard et al, supra.); the data were normalized. The results of these experiments are presented in Figure 30 and Figure 31. Both Gal/Rei h1B4 and m1B4 produced congruent sigmoidal inhibition curves with nearly equivalent IC₅₀s (4-8 nM) that were not significantly different by Students' unpaired t-test. The OKM-1 control IgG did not inhibit PMN attachment. Thus, Gal/Rei hIB4 inhibits adhesion of activated hPMNs to human umbilical vein endothelial cell monolayers to the same extent as native mIB4 in a quantitative homotypic in vitro adhesion assay, illustrating anti-inflammatory activity.

### Inhibition of CTL-mediated cytolysis

Cytotoxic T-lymphocyte (CTL) directed cell killing is an important component of graft rejection following tissue or organ transplantation. Since attachment to and killing of target cells is a CD18-dependent intercellular adhesive event, we determined whether Gal/REI h1B4 inhibits human CTL-mediated cell lysis. Human Q-31 CTL cells were cultivated in RPMI 1640 supplemented with 10% bovine calf serum and 30 units/ml recombinant human IL-2. To induce the differentiated state, fragments of irradiated JY human lymphoblastoid cells were added to the media for 6-7d. The JY cells were propagated as above except without IL-2, and also served as targets for the Q-31 cells. To compare the effects of m1B4 and Gal/Rei h1B4 on cell killing, Q-31 cells were incubated in media with various antibody concentrations for 30 min. at 25 °C before addition of the target cells. To quantify cytolysis, JY target cells were labeled with ⁵¹Cr and mixed with effector cells at various E:T ratios of 8:1 to 2.5:1 at 37 °C. After 4h, the percent of ⁵¹Cr liberated into the culture medium for each concentration of antibodies was determined (in triplicate) as an index of cell killing. Cell killing curves that were generated simultaneously with various concentrations of m1B4 (mOKM-l control) or Gal/Rei h1B4 (hIgG4 control) were utilized to calculate IC₅₀s (Figure 31). Both Gal/Rei h1B4 and m1B4 inhibited JY cell lysis to the same extent. In each case, the mean 1C₅₀ was equal to about 2 nM 1B4, and the inhibition curves for both antibodies were superimposable. These results indicate that Gal/Rei 1B4 can prevent the rejection of transplanted tissues and organs.

### Tissue and Cellular Specificity of Gal/Rei HhB4

The process of humanization might engender abnormal binding properties that could cause h1B4 to associate with and accumulate in unexpected sites in tissues, cells, and their organelles, with toxic consequences. To ascertain whether the binding properties of Gal/Rei h1B4 were altered, we compared the immunofluorescence microscopic (IF) and immunoelectron microscopic (IEM) localization of Gal/Rei h1B4 and native m1B4 in various rabbit tissues, and in human PMNs, U-937 cells, and fibroblasts.

### IF Staining of Tissues and Cells

Healthy 2 kg male New Zealand white rabbits were euthanized, and approxinately 1.0 x 1.0 x 0.5 cm³ tissue blocks were excised, immersed in OCT mounting medium (Miles), and frozen rapidly in liquid nitrogen-cooled Freon 22 (Dupont) at ~-150 °C. Samples were obtained from the following organs: bone marrow, cerebrum, kidney, large intestine, liver, lungs, lymph nodes, myocardium, stomach, striated muscle (leg), and spleen, and stored at -80°C. On the day of an experiment, 5 µm frozen tissue sections were cut with a cryostat at -20 °C, placed on poly-L-lysine-coated glass slides, and air-dried at 25 °C. The sections were immediately immunostained without fixation to avoid denaturation of CD18 antigens. In order to inhibit non-specific binding, slides were washed in 0.1 M Tris-HCl buffer (pH 7.8), and incubated with the clarified supernatant of a solution of 5% non-fat dry milk (Carnation) in 0.1% BSA, 0.1%, NaN₃, and 0.1 M phosphate buffer, pH 7.8 for 1 h at 25 °C. All subsequent staining steps were also conducted for 1 h at 25 °C with intermittent washes in 0.1 M Tris-HCl (pH 7.8). For single-labeling experiments, the sections were stained with a 20 µg/ml solution of primary antibody (mIB4, Gal/Rei hIB4, or hIgG4 control) in staining buffer [0.1% non-fat dry milk, 0.1% BSA, 0.1%, NaN₃, and 0.1 M phosphate buffer (pH 7.8)]. Bound antibodies were detected indirectly with a 25 µg/ml solution of fluorescein isothiocyanate-conjugated affinity-purified goat anti-mouse IgG, or goat anti-human IgG FITC conjugate (Kirkegaard and Perry, Inc.) in staining buffer. In double-staining experiments, specimens were immunolabeled with a mixture of primary antibodies (1 µg/ml m1B4 and 1 µg/ml h1B4 in staining buffer centrifuged at 12,000xg for 15 min.), followed by a clarified mixed-antibody detection solution [25 µg/ml fluorescein isothiocyanate-conjugated affinity-purified goat anti-human IgG and 25 µg/ml rhodamine isothiocyanate-conjugated affinity-purified goat anti-mouse IgG (Kirkegaard and Perry, Inc.) in staining buffer]. Controls for the dual-labeling experiments were clarified solutions of mixed m1B4 plus hIgG4 (1 µg/ml of each antibody), or m1B4, Gal/Rei h1B4, and hIgG4 dissolved alone at 1 1g/ml IgG in staining buffer; IgGs were localized on the sections with the mixed-antibody detection solution described above. Coverslips were mounted on the slides with a solution of 5% n-propyl gallate in 90% glycerol and 10% 1.0 M Na-bicarbonate, and the sections studied with a Zeiss Photomicroscope III equipped with epifluorescence illumination and fluorescein & rhodamine interference filter combinations. Photomicrographs were taken at 16x or 40x with Zeiss neofluar oil-immersion objective lenses using Ilford HP-5 high-speed film at speeds of 1600-6300 ASA.

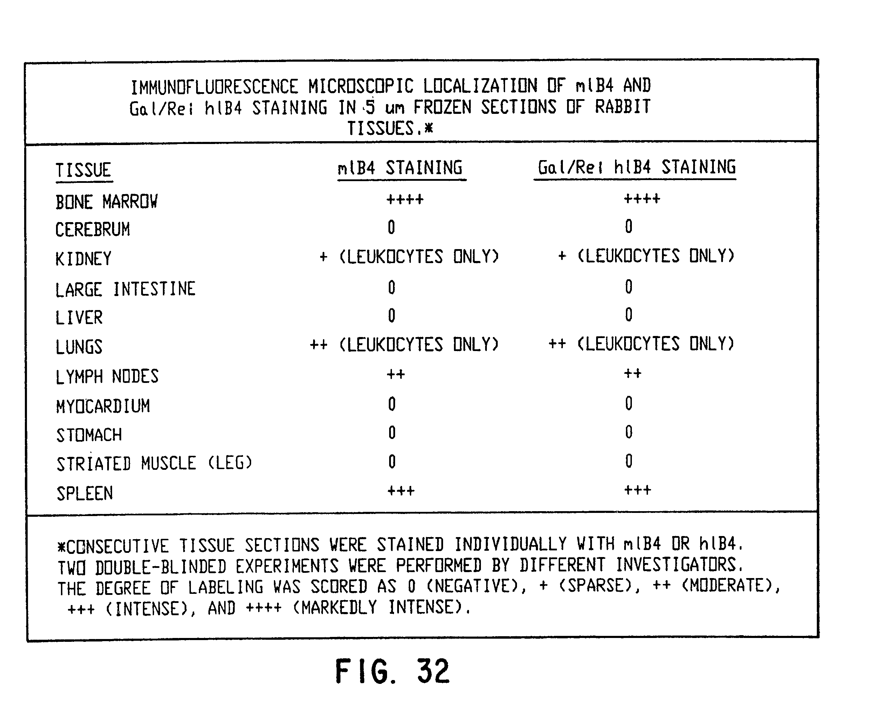

The IF staining patterns of Gal/Rei h1B4 and m1B4 in rabbits are summarized in Figure 32. Specific CD18-positive IF labeling for both recombinant and native IB4 IgGs was observed in tissues known to contain leukocytes. There was no detectable difference in IF distribution or intensity observed with Gal/Rei h1B4 versus m1B4, and control tissues treated with hIgG4 or buffer were always negative. By far, sections of bone marrow presented the most intense CD18 staining with either species of IB4; 79% of these cells exhibited cytoplasmic labeling. Leukocytes of the spleen and the lymph nodes were stained more irregularly and with lower intensity. A conspicuous population of resident leukocytes was detected in the lungs, and to a much lesser extent in kidney glomeruli. Surprisingly, no CD18 staining was seen in the microglial cells of the cerebrum or in the Kupffer cells of the liver. The other tissues were completely unstained. Titration of the primary antibody solution indicated that a 1.0 µg/ml solution of hIB4 or mIB4 was the minimum concentration of either antibody required to obtain maximum IF staining of bone marrow sections.

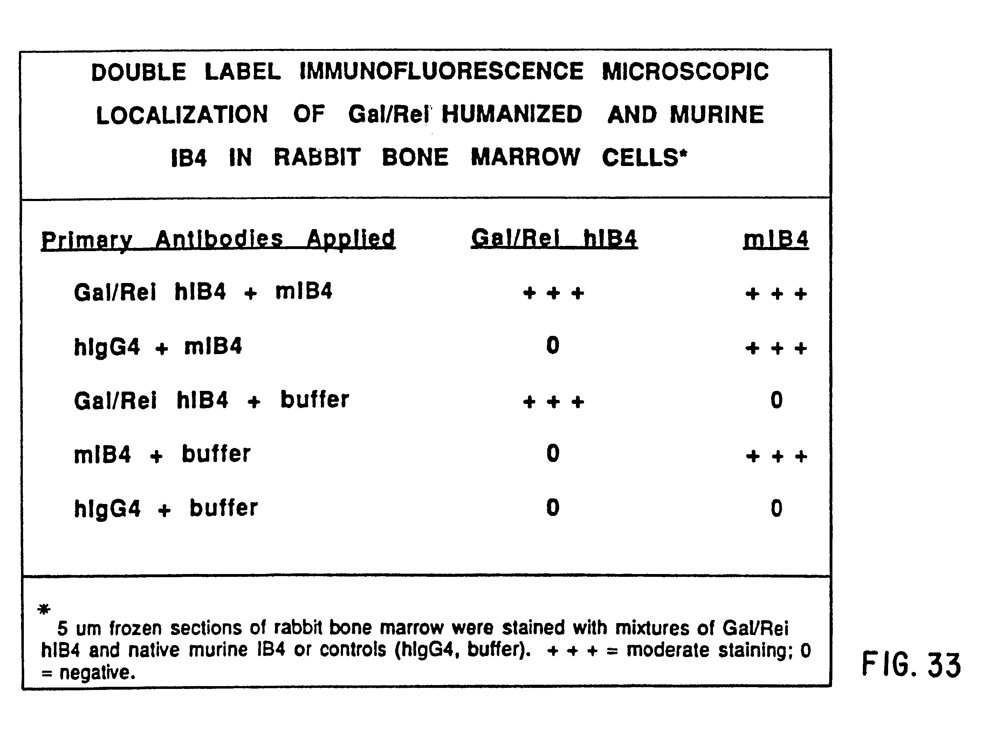

Dual IF staining experiments were conducted to determine whether the antigens recognized by Gal/Rei h1B4 and m1B4 are colocalized in the same cells. Cryosections of bone marrow, spleen, or lymph node were double-labeled with mixtures of Gal/Rei h1B4 and m1B4. As illustrated in Figure 33 for bone marrow, every cell that was positively stained with mIB4 was also labeled with Gal/Rei h1B4. In the control groups, Gal/Rei hIB4 staining (detected under fluorescein optics) was specifically eliminated by substituting hIgG4 for h1B4 in the primary antibody mixture, while retaining the m1B4 labeling (visualized with rhodamine filters). With the converse control, removal of m1B4 from the mixture of primary antibodies ablated the rhodamine labeling, but had no effect on the fluorescein staining generated by Gal/Rei h1B4. These 1B4-colocalization results were therefore highly specific

Thsee data indicate that native and Gal/Rei humanized 1B4 were localized in the same cells (leukocytes) and exhibited identical staining specificity and intensity in various rabbit tissues. The highest levels of CD18 labeling were observed in those tissues which contain large numbers of leukocytes, with the bone marrow presenting the most intense staining. Therefore, our humanization process has not altered the specificity of 1B4 IgG detectable at the light microscopic level of resolution.

### IEM Staining of Human Cell Organelles.

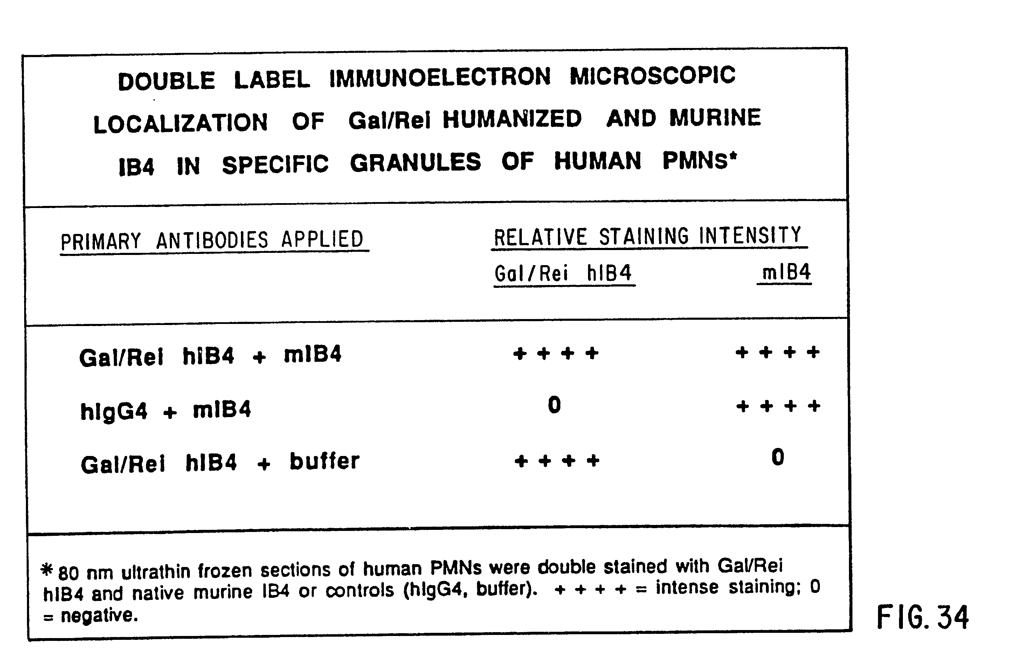

Double label immunoelectron microscopic experiments were conducted to compare the specificity of Gal/Rei h1B4 and m1B4 at the subcellular/supramolecular level of resolution. CD18 antigens have been localized to the specific granules of hPMNs and monocytes via IEM with 60.3 (another Mab that recognizes CD18; Singer et al., J. Cell Biol, 109: 3169-3182 [1989]). Therefore, we determined whether Gal/Rei h1B4 and mIB4 were codistributed in these granules. Human PMNs were isolated from venous blood as described above and prepared for IEM via a modification of a published method (Singer et al, supra). Briefly, the PMNs were fixed with a solution of 3.5% paraformaldehyde and 0.05% glutaraldehyde in 0.1M Na-cacodylate (pH 7.2), 0.1M sucrose, and a mixture of broad spectrum protease inhibitors. Fixation was perfomed under microwave irradiation until the cells reached 45°C (-45 sec.), followed by quenching with excess buffer at 4°C. Cell pellets were embedded in 7% acrylamide, infiltrated with 2.3M sucrose in 0.1M phosphate (pH 7.2), frozen in liquid propane (-190°C) and cut into ultrathin (~80 nm) cryosections. The specimens were double labeled with Gal/Rei h1B4 and m1B4 using 5 nm and 10 nm protain-A colloidal gold conjugates (Janssen Life Science Products) as described, and analyzed at 29,000x with a JEOL 100CX transmission electron microscope. A summary of the immunostaining results for PMNs is shown in Figure34. Both Gal/Rei h1B4 and m1B4 were colocalized in specific granules; negative controls showed that the colloidal gold probes were not cross-reacting nonspecificly. Further, Gal/Rei h1B4 and m1B4 were also colocalized within a population of cytoplasmic granules in U-937 cells (a human myelomonocytic line), but not in human lung fibroblasts (IMR-90). These observations strongly suggest that the binding specificity of Gal/Rei IB4 is comparable to that of mIB4 at supramolecular resolution.

### EXAMPLE 5

### In Vivo Activity Of Recombinant Human Anti-CD18 Antibodies

The in vivo potencies of murine 1B4 (m1B4) and humanized 1B4 (hIB4) (Examples 2 and 4) were compared in the rabbit by assessing their ability to inhibit dermal inflammation, manifest as PMN accumulation and plasma extravasation, elicited by intradermal administration of C5a.

The dorsal hair of female New Zealand White rabbits (2 -2.5 kg) was shaved at least 24 hours prior to experimentation. Rabbits were anesthetized with an intramuscular injection of Ketamine HCI (60 mg) and Xylazine (5 mg).[ ¹²⁵I]-Bovine serum albumin (10 µCi) was injected into the marginal ear vein, as a marker of plasma extravasation. Groups of animals were then treated with saline, m1B4 administered intravenously at 0.07, 0.21 or 0.7 mg/kg, or h1B4 administered intravenously at 0.1, 0.3 or 1 mg/kg 15 minutes before initiation of the dermal inflammation. Thereafter, human recombinant C5a (100 pmol), or saline, in a volume of 50 µl was injected intradermally into 4 replicate sites in the dorsum. Three hours later, a blood sample (1 ml) was taken and centrifuged (8000g; 3 min; 20°C) to prepare cell-free plasma which was aspirated and retained. Animals were then euthanatized with approximately 750 µl Socumb (Sodium Pentobarbital 389 mg/ml in 40% isopropyl alcohol), and injection sites were excised using a 6 mm biopsy punch. Radioactivity ([¹²⁵I]) present in skin samples and cell-free plasma (50 µl) was quantified using a gamma counter. By reference to the specific radioactivity of the cell-free plasma, the extent of plasma extravasation was expressed as µl plasma equivalents per 6 mm biopsy. The skin biopsy was then homogenized in 5 ml of 0.5% Hexadecyltrimethyl ammonium bromide (HTAB) using a polytron homogenizer. Chloroform (1 ml) was added to the sample, which was vortexed and centrifuged (1600g; 15 min.; 20°C). Four aliquots (50 µl) of the aqueous supernatant were added to wells in a 96 well plate for measurement of myeloperoxidase (MPO) activity, as an index of PMN content. Duplicate wells of the 96 well plate received 200 ml buffer (KH₂PO₄ 44 mM; K₂HPO₄ 6 mM; H₂O₂ 0.0015%; pH 6.0) alone (background) and duplicate wells received buffer containing MPO substrate (3′,3-Dimethoxybenzidine dihydrochloride; 360 µg/ml). Reactions were allowed to proceed for 15 min. at room temperature, and MPO activity was measured as the change in absorbance at 450 nm measured in a plate reading spectrophotometer. By reference to a standard curve constructed using known quantities of rabbit PMN in HTAB, the extent of PMN accumulation in each skin biopsy was estimated.

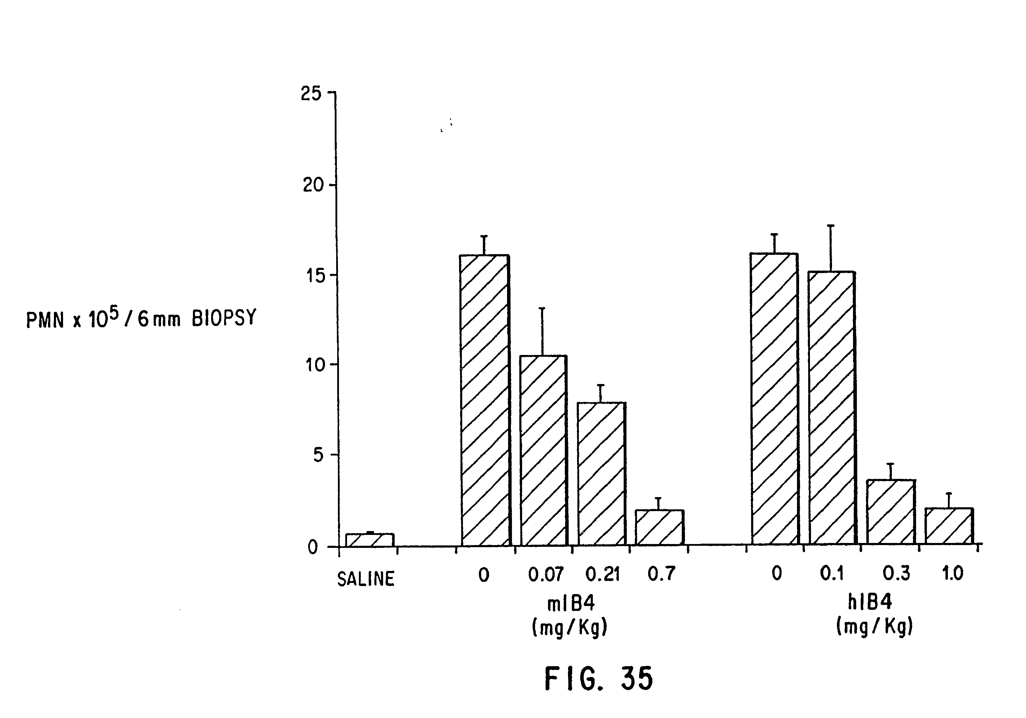

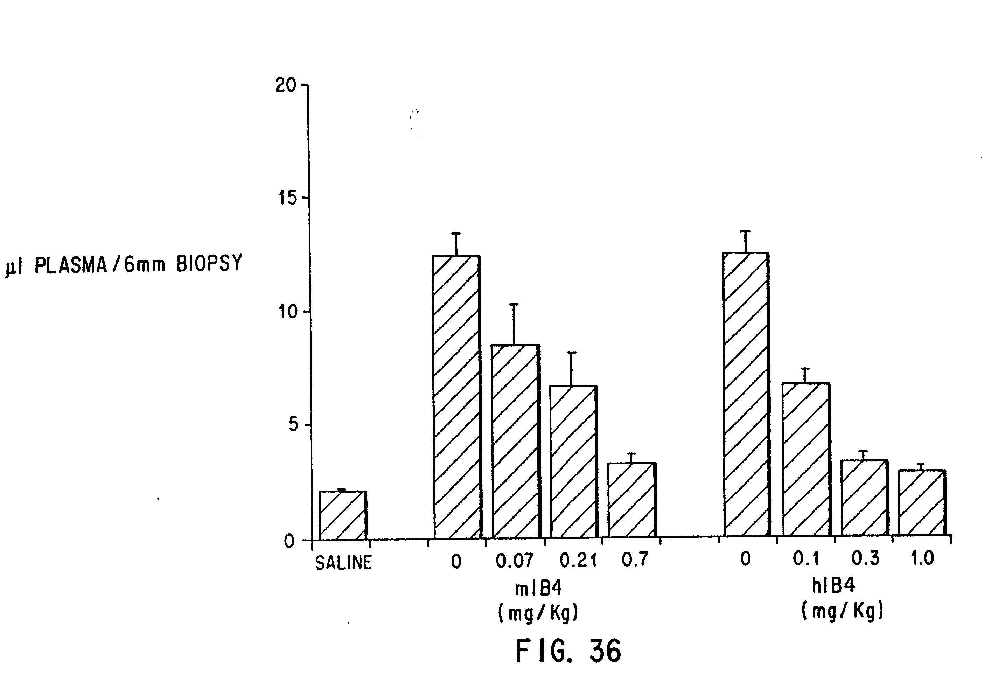

The injection of C5a into the skin of rabbits pretreated with saline produced significant increases in PMN accumulation (Figure 35) and plasma extravasation (Figure 36) compared with skin sites injected with saline. In animals pretreated with either m1B4 or h1B4 there was dose-related inhibition of both PMN accumulation (Figure 35) and plasma extravasation (Figure 36). Both antibodies were of comparable potency, as indicated by the estimated ED₅₀ values for inhibition of PMN accumulation and plasma extravasation which were approximately 0.15 mg/kg for both m1B4 and h1B4.

## Claims

1. A method for producing a humanized recombinant immunoglobulin comprising:
a. preparing polymerase chain reaction primers to amplify the variable portion of the light and heavy chain of a murine antibody which binds to a predefined antigen;
b. using the primers to amplify the variable portions of both heavy and light chains and sequencing the resulting nucleotide chains;
c. determining the murine complimentary determining regions of the heavy and light chains;
d. selecting human variable heavy and light chain frameworks which show a high degree of amino acid similarity with the variable heavy and light chain framework of the murine immunoglobulin;
e. selecting human constant heavy and light chain frameworks;
f. grafting the murine complimentary determining regions of section c to the human framework regions of section e;
g. incorporating the complete DNA sequence for the humanized recombinant immunoglobulin into an appropriate expression vector;
h. transfecting host cells with the expression vector of section g;
i. growing the transfected cells of section g in an environment in which the humanized recombinant immunoglobulin will be produced by the transfected cell;
j. collecting the immunoglobulin.

2. The method of claim 1, section d wherein the selection process will include one or more of the following steps:
a. comparing the framework sequences of the murine monoclonal antibody from which the CDRs have been remeoved with all human heavy chain variable and light chain variable framework sequences;
b. closely comparing the non-surface exposed residues;
c. comparing the tertiary and quanternary structural model of human framework sequences with CDRs in place for comparison with models of the original animal monclonal antibody;
d. screening of human genomic DNA with DNA probes corresponding to framework sequences in chosen animal monoclonal antibody.

3. A polymerase chain reaction method for the simultaneous synthesis and assembly of at least four deoxyoligonucleotides.
